(19) Europäisches Patentamt
European Patent Office
Office européen des brevets

(11) EP 3 818 138 B1

(12) EUROPEAN PATENT SPECIFICATION

(45) Date of publication and mention of the grant of the patent:
**14.05.2025 Bulletin 2025/20**

(21) Application number: **19734410.4**

(22) Date of filing: **02.07.2019**

(51) International Patent Classification (IPC):
***C11D 3/386*** *(2006.01)* ***C12N 9/24*** *(2006.01)*

(52) Cooperative Patent Classification (CPC):
**C11D 3/38636; C11D 3/38609; C11D 3/38618; C11D 3/38645; C12N 9/24; C12Y 302/01052**

(86) International application number:
**PCT/EP2019/067763**

(87) International publication number:
**WO 2020/007875 (09.01.2020 Gazette 2020/02)**

# (54) CLEANING COMPOSITIONS AND USES THEREOF

ENZYMHALTIGE REINIGUNGSZUSAMMENSETZUNGEN UND VERWENDUNGEN DAVON

COMPOSITIONS DE NETTOYAGE ENZYMATIQUES ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.07.2018 EP 18181490**

(43) Date of publication of application:
**12.05.2021 Bulletin 2021/19**

(73) Proprietor: **Henkel AG & Co. KGaA**
**40589 Düsseldorf (DE)**

(72) Inventors:
- **MONRAD, Rune Nygaard**
  **2880 Bagsvaerd (DK)**
- **VEJBORG, Rebecca Munk**
  **2880 Bagsvaerd (DK)**
- **SALOMON, Jesper**
  **2880 Bagsvaerd (DK)**
- **SEGURA, Dorotea Raventos**
  **2880 Bagsvaerd (DK)**

(56) References cited:
**EP-A1- 3 088 506** **WO-A1-2018/002194**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 818 138 B1

## Description

### Reference to a Sequence Listing

This application contains a Sequence Listing in computer readable form, which is incorporated herein by reference.

### Background of the Invention

The present invention relates to compositions such as cleaning compositions comprising a mix of enzymes. The invention further relates, to the use of compositions comprising such enzymes in cleaning processes and/or for cleaning of items such as textiles as well as methods for removal or reduction of organic stains.

### Description of the Related Art

Enzymes have been used in detergents for decades. Usually a cocktail of various enzymes is added to detergent compositions. The enzyme cocktail often comprises various enzymes, each targeting a specific substrate e.g. amylases are active towards starch stains, proteases on protein stains and so forth. Textiles surface and hard surfaces, such as dishes or the inner space of a laundry machine enduring several wash cycles, become soiled with many different types of soiling which may compose of proteins, grease, starch etc. One type of soiling may be organic matter, such as polysaccharides e.g. PNAG (poly-N-acetylglucosamine) and proteins. Some stains may be sticky or gluing, which when present on textile, attracts soils and may cause redeposition or back-staining of soil resulting in a greying of the textile. Additionally, organic stains often cause malodor issue as various malodor molecules can be adhered by the polysaccharides and proteins. There is still a need for cleaning compositions, which effectively prevent, reduce or remove components of organic stains such as polysaccharides. The present invention provides new compositions fulfilling such need.

EP 3088506 disclose detergent with nuclease enzyme. WO 2018/002194 discloses detergent for removal of biofilm.

### Summary of the Invention

The invention relates to a cleaning composition comprising a dispersin, at least one carbohydrase and a cleaning component, wherein the dispersin is a polypeptide having hexosaminidase activity and at least 60% sequence identity to the polypeptide of SEQ ID NO: 17, and wherein the carbohydrase is an alpha-amylase. The invention further relates to the use of such a cleaning composition for cleaning of an item, wherein the item is a textile or a surface. The invention further relates to methods of formulating such a cleaning composition comprising adding a dispersin, a carbohydrase and at least one cleaning component. The invention further relates to a kit intended for deep cleaning, wherein the kit comprises a solution of an enzyme mixture comprising a dispersin and a carbohydrase, wherein the dispersin is a polypeptide having hexosaminidase activity and at least 60% sequence identity to the polypeptide of SEQ ID NO: 17, and wherein the carbohydrase is an alpha-amylase. The invention further relates to a method of deep cleaning of an item, comprising the steps of:

a) contacting the item with such a cleaning composition; and optionally
b) rinsing the item, wherein the item is preferably a textile.

### Detailed Description of the Invention

Various enzymes are applied in cleaning processes each targeting specific types of soiling such as protein, starch and grease soiling. Enzymes are now standard ingredients in detergents for laundry and dish wash. The effectiveness of these commercial enzymes provides detergents which removes much of the soiling. However, organic stains such as biofilm, comprising polysaccharides (exopolysaccharides) and proteins, lipids and other organic substances may be sticky or gluing, which when present on textile, may give rise to redeposition or back-staining of soil resulting in a greying of the textile. Another potential drawback of organic stains is the malodor. Further, when dirty laundry items are washed together with less dirty laundry items the dirt present in the wash liquor tend to stick to organic stains as a result, hereof the laundry item is more "soiled" after wash than before wash. This effect may also be termed re-deposition. Rather than being simple stains, many organic stains comprise multiple different types of soils, such as those derived from biofilm and biofilm EPS (extracellular polymeric substances), body soiling such as sweat, skin cell debris, sebum, textile finishing such as ironing starch, and environmental soils such as dust and dirt. Some types of soil may also attract other types of soil, to form complex mixtures. Such complex stains are difficult to remove completely. It’s not evident from the outset which enzymes

or enzyme mixtures will provide increased or even synergistic removal of complex stains. Some enzymes may negatively impact other enzymes and some enzymes are not stable in detergents or is not stable under laundry conditions. The substances present in e.g. biofilm may be hidden or not accessible for the enzymes. Thus, enzyme blends are needed, which effectively remove such stains. The enzyme combinations of the present invention show effective removal of complex organic stains, involving but excluding biofilm staining or biofilm related staining. The dispersin component of the compositions of the invention provide effective removal of polysaccharide stains in particular poly-N-acetylglucosamine commonly abbreviated PNAG. Without being bound by theory it's assumed that the removal or reduction of the PNAG component of biofilm opens the structure making the stain more accessible to other stain removal enzymes such as carbohydrase. This combined action of the enzymes is surprising, and a result of the stains targeted by the compositions of the invention being particularly complex and difficult to remove using standard detergents. Commercial detergents currently available on the market do not target these complex stains. Such detergents target simple stains primarily composed of one component such as starch and protein. Exopolysaccharides are considered to play an important structural and functional role in the development and maintenance of microbial biofilm. Biofilm are often embedded within an extracellular polymeric substance (EPS). This matrix is a heterogeneous substance which may be composed of various substances such as extracellular DNA, proteins, lipids, and exopolysaccharides such as galactomannan and glycan. Exopolysaccharides are considered as core components of the extracellular matrix of most biofilm. The application of biofilm (component) reducing enzymes holds great potential for application in detergents and laundry either alone, or in particular in combination with conventional detergent enzymes such as the carbohydrases selected from mannanases, cellulases, xyloglucanases and amylases.

Cellulases are enzymes that digest cellulose. Mannanases are mannan-degrading enzymes and includes a and b-mannanases, b-mannosidases and b-glucosidases. Amylases are glycoside hydrolases acting on a-1 ,4-glycosidic bonds and catalyzes the hydrolysis of starch. The combination of standard cleaning enzymes with an enzyme specifically targeting EPS polysaccharides such as PNAG is new. Though, standard detergent may be described as having enzymes such as amylases present, any specific combinations with the PNAG reducing enzymes dispersins is not suggested in the art.

The compositions of the invention comprise a blend of dispersin and a carbohydrase, wherein the dispersin is a polypeptide having hexosaminidase activity and at least 60% sequence identity to the polypeptide of SEQ ID NO: 17, and wherein the carbohydrase is a an alpha-amylase. The compositions effectively reduce or remove organic components, such as starch and PNAG from surfaces such as textiles and hard surfaces e.g. dishes. Such components may be present in e.g. biofilm or biofilm EPS.

The compositions of the invention comprise a blend of dispersin and a carbohydrase, wherein the dispersin is a polypeptide having hexosaminidase activity and at least 60% sequence identity to the polypeptide of SEQ ID NO: 17, and wherein the carbohydrase is an alpha-amylase, and the compositions provides deep cleaning effect, when applied in e.g. laundry process.

The compositions of the invention comprise a blend of dispersin and a carbohydrase, wherein the dispersin is a polypeptide having hexosaminidase activity and at least 60% sequence identity to the polypeptide of SEQ ID NO: 17, and wherein the carbohydrase is an alpha-amylase, and the compositions effectively reduce or limit redeposition when applied in e.g. laundry process.

The compositions of the invention comprise a blend of dispersin and a carbohydrase, wherein the dispersin is a polypeptide having hexosaminidase activity and at least 60% sequence identity to the polypeptide of SEQ ID NO: 17, and wherein the carbohydrase is an amylase, and the compositions effectively reduce or limit malodor of e.g. textiles or hard surfaces such as dishes.

The compositions of the invention comprise a blend of dispersin and a carbohydrase, wherein the dispersin is a polypeptide having hexosaminidase activity and at least 60% sequence identity to the polypeptide of SEQ ID NO: 17, and wherein the carbohydrase is an amylase, and the compositions improve whiteness of textile.

A composition of the invention is preferably a cleaning composition, the composition of the invention comprises at least one dispersin and at least one a carbohydrase, wherein the dispersin is a polypeptide having hexosaminidase activity and at least 60% sequence identity to the polypeptide of SEQ ID NO: 17, and wherein the carbohydrase an amylase. Examples of useful dispersins and carbohydrases are mentioned below in the sections "Polypeptides having dispersin activity" and "Polypeptides having amylase activity" respectively.

<u>Polypeptides having Hexosaminidase activity (hexosaminidases)</u>

The term hexosaminidase includes "dispersin" and the abbreviation "Dsp", and means a polypeptide having hexosaminidase activity, EC 3.2.1.- that catalyzes the hydrolysis of β-1,6-glycosidic linkages of N-acetyl-glucosamine polymers found e.g. in biofilm. The term hexosaminidase includes polypeptides having N-acetylglucosaminidase activity and β-N-acetylglucosaminidase activity. The term "polypeptide having hexosaminidase activity" may be used interchangeably with the term hexosaminidases and similarly the term "polypeptide having β-N-acetylglucosaminidase

activity" may be used interchangeably with the term β-N-acetylglucosaminidases. For purposes of the present invention, hexosaminidase activity is determined according to the procedure described in Assay I. In a preferred embodiment, the polypeptide having hexosaminidase activity is a dispersin. In a preferred embodiment, the polypeptide having hexosaminidase activity is a β-N-acetylglucosaminidase targeting poly-β-1,6-N-acetylglucosamine.

In one embodiment, the invention relates to a cleaning composition comprising a dispersin, preferably a β-N-acetylglucosaminidase, at least one carbohydrase and a cleaning component, wherein the dispersin is a polypeptide having hexosaminidase activity and at least 60% sequence identity to the polypeptide of SEQ ID NO: 17, and wherein the carbohydrase is an alpha-amylase.

One embodiment of the invention relates to a composition comprising a hexosaminidase, preferably a β-N-acetylglucosaminidase e.g. a dispersin, polypeptide wherein the polypeptide is selected from the group consisting of polypeptides:

a) a polypeptide having at least 60%, e.g., at least 65%, at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 17,

The dispersins above may be combined with any of the carbohydrases below to form a blend to be added to a composition according to the invention, wherein the carbohydrase is an alpha-amylase.

<u>Polypeptides having Carbohydrase activity (Carbohydrases)</u>

Carbohydrase is a protein/enzyme that catalyse carbohydrates to break down carbohydrates to e.g. simple sugar such as monosaccharides. Thus, carbohydrases are any of a group of enzymes that promote hydrolysis of a carbohydrate. Starch hydrolyzing carbohydrases (e.g. amylases) work on e.g. amylose and amylopectin and non-starch carbohydrases includes enzymes which hydrolyze polymers made up of carbon sugars e.g. cellulases which will ultimately produce glucose when complete hydrolysis is achieved. Another example is lactase which hydrolyses lactose to glucose and galactose. Examples of carbohydrases include amylases, cellulases, xyloglucanases and mannanases. The carbohydrases to be incorporated in a composition according to the invention is selected from alpha-amylases. Additionally, the composition might also comprise further preferably selected from xyloglucanases, cellulases, mannanases and amylases.

<u>Polypeptides having Mannanase activity</u>

The term "mannanase" is defined here as an enzyme that hydrolyses compounds known as mannans. Mannanases are enzyme catalyzing hydrolyses of 1,4-beta-D-mannosidic linkages in mannans, galactomannans, glucomannans, and galactoglucomannans. Mannans are polysaccharides with a backbone of β-1,4-linked D-mannopyranosyl residues, which can contain galactose or acetyl substitutions and may have glucose residues in the backbone. The main enzyme type participating in the degradation of mannans are endo-1,4-3-mannanases (EC 3.2.1.78), which hydrolyze the internal glycoside bonds in the mannan backbone. The term "mannanase activity" is defined as an enzyme catalyzed hydrolysis of mannan, for purposes of the present invention, mannanase activity is determined according to the procedure described in the Assay II. A cleaning composition might comprise a dispersin and a mannanase enzyme comprising a polypeptide having mannan endo-1,4-beta-mannosidase activity (EC 3.2.1.78) that catalyzes the hydrolysis of 1,4-beta-D-mannosidic linkages in mannans, galactomannans and/or glucomannans. According to CAZy (www.cazy.org), endo-1,4-3-mannanases can be found in glycoside hydrolase families 5, 26 and 113. Suitable mannanases include those of bacterial or fungal origin. Chemically or genetically modified mannanases are included. The mannanase may be an alkaline mannanase of Family 5 or 26. It may be a wild-type from *Bacillus* or *Humicola,* particularly B. *agaradhaerens, B. licheniformis, B. halodurans, B. clausii,* or *H. insolens.* Commercially available mannanases are Mannaway (Novozymes A/S), and EFFECTENZ™ M1000 from Dupont. Preferred mannanases include the GH5 mannanase obtained from *Bacillus bogoriensis* described in WO1999/064619A cleaning composition might comprise a dispersin and at least one enzyme having mannanase activity, which may be obtained from a bacterial strain of the genus *Bacillus.* Preferred mannanases include the GH5 mannanase obtained from *Bacillus bogoriensis* described in WO1999/064619. Preferably, a polypeptide comprising the amino acid sequence shown in SEQ ID NO: 24. Other preferred mannanases include any of the GH26 Mannanases, mannanase from *Preussia aemulans* mature sequence of SEQ ID NO: 2 of WO2017/021515, mannanase from *Yunnania penicillata* mature sequence of SEQ ID NO: 2 of WO2017/021516, mannanase from *Myrothecium roridum* mature sequence of SEQ ID NO: 2 of WO2017/021517, mannanase from *Chaetomium brasiliense* mature sequence of SEQ ID NO: 2 of WO2017/021518, mannanases from *Ascobolus stictoideus* or mannanase from *Chaetomium virescens* SEQ ID NO: 3 and 6 from WO2015/040159.

A cleaning composition might comprise a dispersin and at least one enzyme classified in the EC 3.2.1.78 and which has mannanase activity.

One preferred cleaning enzyme to be combined with the dispersin enzyme is a mannanase, including polypeptides that are substantially homologous to the polypeptides shown in SEQ ID NO: 24 and species homologs (paralogs or orthologs) thereof.

Other preferred cleaning enzyme to be combined with the dispersin enzyme is a mannanase, including polypeptides that are substantially homologous to the polypeptides shown in SEQ ID NO: 34, 35, 36, 37, 38, 39, 40, 41 or 42 and species homologs (paralogs or orthologs) thereof.

The term "substantially homologous" is used herein to denote polypeptides having at least 50%, at least 55%, at least 60%, at least 65%, preferably at least 70%, more preferably at least 75%, more preferably at least 80%, more preferably at least 85%, more preferably at least 90% more preferably at least 95%, more preferably at least 97%, even more preferably at least 98% sequence identity to the sequence.

Preferred mannanases to be used in compositions according to the invention can be selected from the group consisting of;

a) a mannanase, wherein the mannanase belongs to the Glycoside Hydrolase Family 5 mannanases;

i. a mannanase having at least 55%, at least 58%, at least 60%, at least 65%, at least 70%, at least 75%, at least 80%, at least 85%, at least 90%, at least 95%, at least 98% or 100% sequence identity to SEQ ID NO: 24, preferably from *Bacillus* e.g. *Bacillus bogoriensis* ;

b) a mannanase wherein the mannanase belongs to the Glycoside Hydrolase Family 26 mannanases;

i. a mannanase having at least 60%, at least 65%, at least 70%, at least 75%, at least 80%, at least 85%, at least 90%, at least 95%, at least 98% or 100% sequence identity to SEQ ID NO: 34, preferably obtained from *Paenibacillus woosongensis*;

ii. a mannanase having at least 60%, at least 65%, at least 70%, at least 75%, at least 80%, at least 85%, at least 90%, at least 95%, at least 98% or 100% sequence identity to SEQ ID NO: 35, preferably obtained from *Paenibacillus illinoisensis* ;

iii. a mannanase having at least 60%, at least 65%, at least 70%, at least 75%, at least 80%, at least 85%, at least 90%, at least 95%, at least 98% or 100% sequence identity to SEQ ID NO: 36, preferably obtained from *Neobulgaria* sp.;

iv. a mannanase having at least 60%, at least 65%, at least 70%, at least 75%, at least 80%, at least 85%, at least 90%, at least 95%, at least 98% or 100% sequence identity to SEQ ID NO: 37, preferably obtained from *Preussia aemulans* ;

v. a mannanase having at least 60%, at least 65%, at least 70%, at least 75%, at least 80%, at least 85%, at least 90%, at least 95%, at least 98% or 100% sequence identity to SEQ ID NO: 38, preferably obtained from *Yunnania penicillata,*

vi. a mannanase having at least 60%, at least 65%, at least 70%, at least 75%, at least 80%, at least 85%, at least 90%, at least 95%, at least 98% or 100% sequence identity to SEQ ID NO: 39, preferably obtained from *Myrothecium roridum* ;

vii. a mannanase having at least 60%, at least 65%, at least 70%, at least 75%, at least 80%, at least 85%, at least 90%, at least 95%, at least 98% or 100% sequence identity to SEQ ID NO: 40, preferably obtained from *Chaetomium brasiliense,*

viii. a mannanase having at least 60%, at least 65%, at least 70%, at least 75%, at least 80%, at least 85%, at least 90%, at least 95%, at least 98% or 100% sequence identity to SEQ ID NO: 41 , preferably obtained from *Ascobolus stictoideus,* and

ix. a mannanase having at least 60%, at least 65%, at least 70%, at least 75%, at least 80%, at least 85%, at least 90%, at least 95%, at least 98% or 100% sequence identity to SEQ ID NO: 42, preferably obtained from *Chaetomium virescens.*

Polypeptides having Cellulase activity

The term "cellulase" is defined in the present context as an enzyme that hydrolyses cellulose. In a preferred embodiment of the invention, the cellulase is an endoglucanase. The term "cellulase activity" is defined herein as an enzyme catalyzed hydrolysis of 1,4-beta-D-glucosidic linkages in beta-1,4-glucan (cellulose). For purposes of the present invention, cellulase activity is determined using AZCL-HE-cellulose (from Megazyme) as the reaction substrate, as shown in Assay IV. Suitable cellulases include those of bacterial or fungal origin. Chemically modified or protein engineered

mutants are included. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and WO99/001544.

Other cellulases are endo-beta-1,4-glucanase enzyme having a sequence of at least 97% identity to the amino acid sequence of position 1 to position 773 of SEQ ID NO: 2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Celluzyme™, and Carezyme™ (Novozymes A/S) Carezyme Premium™ (Novozymes A/S), Celluclean ™ (Novozymes A/S), Celluclean Classic™ (Novozymes A/S), Cellusoft™ (Novozymes A/S), Whitezyme™ (Novozymes A/S), Clazinase™, and Puradax HA™ (Genencor International Inc.), and KAC-500(B)™ (Kao Corporation), Revitalenz™ 1000, Revitalenz™ 2000, Revitalenz™ 3000 (Dupont).

A cleaning composition might comprise a dispersin and a polypeptide having cellulase activity, which comprise the amino acid sequence of SEQ ID NO: 25. A cleaning composition might comprise a dispersin and a polypeptide having cellulase activity, which comprises an amino acid sequence having at least 60%, at least 65%, at least 70%, more preferably at least 75%, more preferably at least 80%, more preferably at least 85%, more preferably at least 90%, more preferably at least 95%, more preferably at least 96%, even more preferably at least 97%, most preferably at least 98%, or even most preferably at least 99% or 100% sequence identity to SEQ ID NO: 25.

A cleaning composition might comprise a dispersin and a polypeptide having cellulase activity, which comprise the amino acid sequence of SEQ ID NO: 26. A cleaning composition might comprise a dispersin and a polypeptide having cellulase activity, which comprises an amino acid sequence having at least 60%, at least 65%, at least 70%, more preferably at least 75%, more preferably at least 80%, more preferably at least 85%, more preferably at least 90%, more preferably at least 95%, more preferably at least 96%, even more preferably at least 97%, most preferably at least 98%, or even most preferably at least 99% or 100% sequence identity to SEQ ID NO: 26.

A cleaning composition might comprise a dispersin and a polypeptide having cellulase activity, which comprise the amino acid sequence of SEQ ID NO: 27. A cleaning composition might comprise a dispersin and a polypeptide having cellulase activity, which comprises an amino acid sequence having at least 60%, at least 65%, at least 70%, more preferably at least 75%, more preferably at least 80%, more preferably at least 85%, more preferably at least 90%, more preferably at least 95%, more preferably at least 96%, even more preferably at least 97%, most preferably at least 98%, or even most preferably at least 99% or 100% sequence identity to SEQ ID NO: 27.

A cleaning composition might comprise a dispersin and a polypeptide having cellulase activity, which comprise the amino acid sequence of SEQ ID NO: 28. A cleaning composition might comprise a dispersin and a polypeptide having cellulase activity, which comprises an amino acid sequence having at least 60%, at least 65%, at least 70%, more preferably at least 75%, more preferably at least 80%, more preferably at least 85%, more preferably at least 90%, more preferably at least 95%, more preferably at least 96%, even more preferably at least 97%, most preferably at least 98%, or even most preferably at least 99% or 100% sequence identity to SEQ ID NO: 28.

A cleaning composition can also comprise a carbohydrase, which is a cellulase which also comprises an endoglucanase or xyloglucanase activity, and which hydrolyse beta-1,4-glucosidic bonds in xyloglucan. A polypeptide comprising xyloglucanase activity may be classified as a cellulase or may be classified as a xyloglucanase. The term "xyloglucanase activity" or xyloglucanase is defined herein as an enzyme catalyzed hydrolysis of xyloglucan, which is shown in Assay III. A xyloglucanase can comprise parent xyloglucanase and the variants thereof.

A cleaning composition might comprise a xyloglucanase which is a polypeptide comprising an amino acid sequence of SEQ ID NO: 29. A cleaning composition might comprise a dispersin and a polypeptide, preferably having xyloglucanase activity, which comprises an amino acid sequence having at least 60%, at least 65%, at least 70%, more preferably at least 75%, more preferably at least 80%, more preferably at least 85%, more preferably at least 90%, more preferably at least 95%, more preferably at least 96%, even more preferably at least 97%, most preferably at least 98%, or even most preferably at least 99% or 100% sequence identity to SEQ ID NO: 29.

Polypeptides having Amylase activity

An amylase is an enzyme that hydrolyses starch into sugars, for purposes of the present invention, amylase activity is determined according to the procedure described in one of the Assays under V. Suitable amylases include alpha-amylases and/or a glucoamylases and may be of bacterial or fungal origin. Chemically modified or protein engineered mutants are included. Amylases include, for example, alpha-amylases obtained from *Bacillus,* e.g., a special strain of

*Bacillus licheniformis,* described in more detail in GB 1,296,839.

In one aspect of the present invention, the cleaning composition comprise a dispersin and a polypeptide having amylase activity, which comprise the amino acid sequence of SEQ ID NO: 30. In one aspect of the present invention, the cleaning composition comprise a dispersin and a polypeptide having amylase activity, which comprises an amino acid sequence having at least 60%, at least 65%, at least 70%, more preferably at least 75%, more preferably at least 80%, more preferably at least 85%, more preferably at least 90%, more preferably at least 95%, more preferably at least 96%, even more preferably at least 97%, most preferably at least 98%, even most preferably at least 99% or 100% sequence identity to SEQ ID NO: 30.

In one aspect of the present invention, the cleaning composition comprise a dispersin and a polypeptide having amylase activity, which comprise the amino acid sequence of SEQ ID NO: 31. In one aspect of the present invention, the cleaning composition comprise a dispersin and a polypeptide having amylase activity, which comprises an amino acid sequence having at least 60%, at least 65%, at least 70%, more preferably at least 75%, more preferably at least 80%, more preferably at least 85%, more preferably at least 90%, more preferably at least 95%, more preferably at least 96%, even more preferably at least 97%, most preferably at least 98%, even most preferably at least 99% or 100% sequence identity to SEQ ID NO: 31.

In one aspect of the present invention, the cleaning composition comprise a dispersin and a polypeptide having amylase activity, which comprise the amino acid sequence of SEQ ID NO: 32. In one aspect of the present invention, the cleaning composition comprise a dispersin and a polypeptide having amylase activity, which comprises an amino acid sequence having at least 60%, at least 65%, at least 70%, more preferably at least 75%, more preferably at least 80%, more preferably at least 85%, more preferably at least 90%, more preferably at least 95%, more preferably at least 96%, even more preferably at least 97%, most preferably at least 98%, even most preferably at least 99% or 100% sequence identity to SEQ ID NO: 32.

In one aspect of the present invention, the cleaning composition comprise a dispersin and a polypeptide having amylase activity, which comprise the amino acid sequence of SEQ ID NO: 33. In one aspect of the present invention, the cleaning composition comprise a dispersin and a polypeptide having amylase activity, which comprises an amino acid sequence having at least 60%, at least 65%, at least 70%, more preferably at least 75%, more preferably at least 80%, more preferably at least 85%, more preferably at least 90%, more preferably at least 95%, more preferably at least 96%, even more preferably at least 97%, most preferably at least 98%, even most preferably at least 99% or 100% sequence identity to SEQ ID NO: 33.

In one aspect of the present invention, the cleaning composition comprise a dispersin and a polypeptide having amylase activity, which comprise the amino acid sequence of SEQ ID NO: 43, public available from GENESEQP: BFJ77696. In one aspect of the present invention, the cleaning composition comprise a dispersin and a polypeptide having amylase activity, which comprises an amino acid sequence having at least 60%, at least 65%, at least 70%, more preferably at least 75%, more preferably at least 80%, more preferably at least 85%, more preferably at least 90%, more preferably at least 95%, more preferably at least 96%, even more preferably at least 97%, most preferably at least 98%, even most preferably at least 99% or 100% sequence identity to SEQ ID NO 43.

Additional amylases include amylases comprising the polypeptide shown in SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444. Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193. Other amylases which are suitable are hybrid alpha-amylase comprising residues 1-33 of the alpha-amylase derived from *B. amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: 48, 49, 107, 156, 181, 190, 197, 201, 209 and 264. Most preferred variants of the hybrid alpha-amylase comprising residues 1-33 of the alpha-amylase derived from B. amyloliquefaciens shown in SEQ ID NO: 6 of WO 2006/066594 and residues 36-483 of SEQ ID NO: 4 are those having the substitutions:

M197T;
H156Y+A181T+N190F+A209V+Q264S; or
G48A+T491+G107A+H156Y+A181T+N190F+1201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and

K269. Particularly preferred amylases are those having deletion in positions R181 and G182, or positions H183 and G184. Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.
Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264. Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or
S125A+N128C+T1311+T165I+K178L+T182G+Y305R+G475K wherein the variants are C-terminally truncated and optionally further comprises a substitution at position 243 and/or a deletion at position 180 and/or position 181.

Further suitable amylases are amylases having SEQ ID NO: 1 of WO13184577 or variants having 90% sequence identity to SEQ ID NO: 1 thereof. Preferred variants of SEQ ID NO: 1 are those having a substitution, a deletion or an insertion in one of more of the following positions: K176, R178, G179, T180, G181, E187, N192, M199, I203, S241, R458, T459, D460, G476 and G477. More preferred variants of SEQ ID NO: 1 are those having the substitution in one of more of the following positions: K176L, E187P, N192FYH, M199L, I203YF, S241QADN, R458N, T459S, D460T, G476K and G477K and/or deletion in position R178 and/or S179 or of T180 and/or G181. Most preferred amylase variants of SEQ ID NO: 1 are those having the substitutions:

E187P+1203Y+G476K
E187P+I203Y+R458N+T459S+D460T+G476K,
wherein the variants optionally further comprise a substitution at position 241 and/or a deletion at position 178 and/or position 179.

Further suitable amylases are amylases having SEQ ID NO: 1 of WO10104675 or variants having 90% sequence identity to SEQ ID NO: 1 thereof. Preferred variants of SEQ ID NO: 1 are those having a substitution, a deletion or an insertion in one of more of the following positions: N21, D97, V128 K177, R179, S180, I181, G182, M200, L204, E242, G477 and G478. More preferred variants of SEQ ID NO: 1 are those having the substitution in one of more of the following positions: N21D, D97N, V128I K177L, M200L, L204YF, E242QA, G477K and G478K and/or deletion in position R179 and/or S180 or of I181 and/or G182. Most preferred amylase variants of SEQ ID NO: 1 are those having the substitutions:

N21 D+D97N+V128I
wherein the variants optionally further comprise a substitution at position 200 and/or a deletion at position 180 and/or position 181. Other suitable amylases are the alpha-amylase comprising the polypeptide sequence shown in SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has

substitutions in all these positions. Other examples are amylase variants such as those described in WO2011/098531, WO2013/001078 and WO2013/001087.

Commercially available amylases are Duramyl™, Termamyl™, Fungamyl™, Stainzyme™, Stainzyme Plus™, Natalase™, Liquozyme X and BAN™ (from Novozymes A/S), and Rapidase™, Purastar™/Effectenz™, Powerase, Preferenz S1000, Preferenz S100 and Preferenz S110 (from Genencor International Inc./DuPont).

Compositions

The invention relates to cleaning compositions comprising a dispersin and a carbohydrase in combination with one or more additional cleaning composition components, wherein the dispersin is a polypeptide having hexosaminidase activity and at least 60% sequence identity to the polypeptide of SEQ ID NO: 17, and wherein the carbohydrase is an alpha-amylase. As described the carbohydrases may act synergistically with the dispersin in reduction, and removal of target substrate e.g. the PNAG of biofilm and It's believed that when the dispersins and carbohydrases are acting together, the biofilm and PNAG components are more effectively dispersed or removed. It is thus advantageous to formulate dispersins with carbohydrases such as cellulases, amylases, mannanases and xyloglucanases in cleaning compositions for cleaning. One aspect of the invention relates to a method of formulating a cleaning composition comprising adding a dispersin, at least one carbohydrase and a cleaning component, wherein the carbohydrase is an alpha-amylase. The invention further relates to a kit intended for deep cleaning, wherein the kit comprises a solution of an enzyme mixture comprising a dispersin and a carbohydrase, wherein the carbohydrase is an alpha-amylase.

In one aspect of the invention the carbohydrase is an amylase. In one aspect, the invention relates to a cleaning composition comprising a dispersin, a carbohydrase and a cleaning component, wherein the dispersin is a polypeptide having hexosaminidase activity and at least 60% sequence identity to the polypeptide of SEQ ID NO: 17, and wherein the carbohydrase is an alpha-amylase. In one aspect, the invention relates to a cleaning composition comprising a dispersin, a carbohydrase and a cleaning component, wherein the dispersin is a polypeptide having hexosaminidase activity and at least 60% sequence identity to the polypeptide of SEQ ID NO: 17, and wherein the carbohydrase is an alpha-amylase, preferably selected from the group consisting of a polypeptide having at least 60%, at least 65%, at least 70%, at least 75%, at least 80%, at least 85%, at least 90%, at least 95%, at least 98% or 100% sequence identity to the polypeptide shown in SEQ ID NO: 30, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 31, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 32, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 33, and a polypeptide having at least 60%, at least 65%, at least 70%, at least 75%, at least 80%, at least 85%, at least 90%, at least 95%, at least 98% or 100% sequence identity to the polypeptide shown in SEQ ID NO: 43.

The dispersins to be formulated together with the carbohydrases or to be used together with the carbohydrases should be compatible with cleaning components. dispersins are at present not standard ingredients in cleaning compositions. However, the applicant has identified dispersins suitable for use in cleaning compositions e.g. in WO 2017/186936, WO 2017/186937 and WO 2017/186943. These applications also mentioned that dispersins may be formulated with other enzymes e.g. carbohydrases. However, none of these applications indicate that the dispersins may have synergy with e.g. mannanases, xyloglucanases, amylases or cellulases. Enzymes, such as dispersins should not only be compatible with the cleaning components, the dispersins should also be compatible with other enzymes, which may be present in a typical cleaning composition. Surprisingly, it was found that carbohydrases such as alpha-amylases not only are compatible but may even act synergistically in respect of complex stain such as biofilm and polysaccharide reduction and removal e.g. in cleaning. The combination of dispersin and alpha-amylase provide an additive or even a synergistic effect defined as an effect over and above the sum of the effects of the enzymes taken individually. Example 1 of the application demonstrate a synergy effect when a carbohydrase exemplified with a selection of amylases and dispersin are used in combination. The effect is measured as remission ($REM^{460nm}$) values and the wash performance synergies, is calculated as $WP_{synergy}$ ($\Delta REM^{460nm}{}_{(combination\ of\ enzymes)}$ - $\Delta REM^{460nm}{}_{(sum\ of\ individual\ enzyme}$ treatments)), where delta values are measured as ($REM460nm_{(swatches\ washed\ with}$ enzyme) - $REM460nM_{(swatches\ washed\ without\ enzyme)}$).

In one embodiment the combination of dispersin and amylase provide an additive or even a synergistic effect defined as an effect over and above the sum of the effects of the enzymes taken individually, when measured as described in Example 1, wherein the effect is measured as remission ($REM^{460nm}$) values and the wash performance synergies, is calculated as $WP_{synergy}$ ($\Delta REM^{460nm}{}_{(dispersin\ +\ amylase)}$ - $\Delta REM^{460nm}{}_{(sum\ of\ individual\ enzyme\ treatments)}$), where delta values are measured as ($REM460nm_{(swatches\ washed\ with\ amylase\ or\ dispersin\ enzyme)}$ - $REM460nm_{(swatches\ washed\ without\ enzyme)}$).

Particularly useful dispersins may be those of microbial origin. One embodiment of the invention relates to a cleaning composition comprising a dispersin, a carbohydrase and at least one cleaning component, wherein the dispersin

is a polypeptide having hexosaminidase activity and at least 60% sequence identity to the polypeptide of SEQ ID NO: 17, wherein the dispersin is obtained from *Terribacillus,* and wherein the carbohydrase is an alpha-amylase.

One embodiment of the invention relates to a cleaning composition comprising a dispersin, at least one carbohydrase and a cleaning component, wherein the carbohydrase is an alpha-amylase, and wherein the dispersin has at least 60%, at least 65%, at least 70%, at least 75%, at least 80%, at least 85%, at least 90%, at least 95%, at least 98% or 100% sequence identity to the amino acid sequence shown in SEQ ID NO: 17.

One embodiment relates to a cleaning composition comprising a *Terribacillus* dispersin, an alpha-amylase and at least one cleaning component, wherein the alpha-amylase is selected from the group consisting of a polypeptide having at least 60%, at least 65%, at least 70%, at least 75%, at least 80%, at least 85%, at least 90%, at least 95%, at least 98% or 100% sequence identity to the polypeptide shown in SEQ ID NO: 30, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 31, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 32, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 33, and a polypeptide having at least 60%, at least 65%, at least 70%, at least 75%, at least 80%, at least 85%, at least 90%, at least 95%, at least 98% or 100% sequence identity to the polypeptide shown in SEQ ID NO: 43.

One embodiment relates to a cleaning composition comprising a dispersin, an alpha-amylase and at least one cleaning component, wherein the alpha-amylase is selected from the group consisting of a polypeptide having at least 60%, at least 65%, at least 70%, at least 75%, at least 80%, at least 85%, at least 90%, at least 95%, at least 98% or 100% sequence identity to the polypeptide shown in SEQ ID NO: 30, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 31, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 32, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 33, and a polypeptide having at least 60%, at least 65%, at least 70%, at least 75%, at least 80%, at least 85%, at least 90%, at least 95%, at least 98% or 100% sequence identity to the polypeptide shown in SEQ ID NO: 43 and wherein the dispersin has at least 60%, at least 65%, at least 70%, at least 75%, at least 80%, at least 85%, at least 90%, at least 95%, at least 98% or 100% sequence identity to the amino acid sequence shown in SEQ ID NO: 17.

One embodiment of the invention relates to a composition comprising

a) at least 0.001 ppm e.g. 0.1 ppm or 1 ppm of at least one polypeptide having hexosaminidase activity, wherein the polypeptide is selected for the group consisting of:
xvi) a polypeptide having hexosaminidase activity selected from: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17; and
b) at least 0.001 ppm e.g. 0.1 ppm or 1 ppm of one or more carbohydrase, wherein the carbohydrase is selected from the group consisting of;

v. an alpha-amylase selected from a polypeptide having at least 60%, at least 65%, at least 70%, at least 75%, at least 80%, at least 85%, at least 90%, at least 95%, at least 98% or 100% sequence identity to the polypeptide shown in SEQ ID NO: 30, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 31, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 32, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 33 and a polypeptide having at least 60%, at least 65%, at least 70%, at least 75%, at least 80%, at least 85%, at least 90%, at least 95%, at least 98% or 100% sequence identity to the polypeptide shown in SEQ ID NO: 43;

and
c) at least one additional component e.g. cleaning component, preferably selected from surfactants, builders, bleach components, polymers and dispersing agents.

The carbohydrase and dispersin may be included in the cleaning composition of the present invention at a level of from 0.01 to 1000 ppm, from 1 ppm to 1000 ppm, from 10 ppm to 1000 ppm, from 50 ppm to 1000 ppm, from 100 ppm to

1000 ppm, from 150 ppm to 1000 ppm, from 200 ppm to 1000 ppm, from 250 ppm to 1000 ppm, from 250 ppm to 750 ppm, from 250 ppm to 500 ppm. The dispersins above may be combined with carbohydrases to form a blend to be added to the wash liquor solution according to the invention. The concentration of the dispersin in the wash liquor solution is typically in the range of wash liquor from 0.00001 ppm to 10 ppm, from 0.00002 ppm to 10 ppm, from 0.0001 ppm to 10 ppm, from 0.0002 ppm to 10 ppm, from 0.001 ppm to 10 ppm, from 0.002 ppm to 10 ppm, from 0.01 ppm to 10 ppm, from 0.02 ppm to 10 ppm, 0.1 ppm to 10 ppm, from 0.2 ppm to 10 ppm, from 0.5 ppm to 5 ppm. The concentration of the carbohydrases in the wash liquor solution is typically in the range of wash liquor from 0.00001ppm to 10 ppm, from 0.00002 ppm to 10 ppm, from 0.0001 ppm to 10 ppm, from 0.0002 ppm to 10 ppm, from 0.001 ppm to 10 ppm, from 0.002 ppm to 10 ppm, from 0.01 ppm to 10 ppm, from 0.02 ppm to 10 ppm, 0.1 ppm to 10 ppm, from 0.2 ppm to 10 ppm, from 0.5 ppm to 5 ppm. The dispersins may be combined with any of the carbohydrases below to form a blend to be added to a composition according to the invention.

One embodiment relates to a cleaning composition comprising a dispersin, a carbohydrase and at least one cleaning component, wherein the amount of dispersin in the composition is from 0.01 to 1000 ppm and the amount of carbohydrase is from 0.01 to 1000 ppm.

The invention relates to cleaning compositions comprising an enzyme combination of the present invention in combination with one or more additional cleaning composition component(s). The choice of additional components is within the skill of the artisan and includes conventional ingredients, including the exemplary non-limiting components set forth below.

The choice of cleaning components may include, for textile care, the consideration of the type of textile to be cleaned, the type and/or degree of soiling, the temperature at which cleaning is to take place, and the formulation of the detergent product. Although components mentioned below are categorized by general header according to a functionality, this is not to be construed as a limitation, as a component may comprise additional functionalities as will be appreciated by the skilled artisan.

Surfactants

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 0.1% to about 15%, such as about 1% to about 40%, or about 3% to about 20%, or about 3% to about 10%. The surfactant(s) is chosen based on the desired cleaning application, and may include any conventional surfactant(s) known in the art. When included therein the detergent will usually contain from about 1% to about 40% by weight of an anionic surfactant, such as from about 5% to about 30%, including from about 5% to about 15%, or from about 15% to about 20%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof.

When included therein the detergent will usually contain from about 1% to about 40% by weigh of a cationic surfactant, for example from about 0.5% to about 30%, in particular from about 1% to about 20%, from about 3% to about 10%, such as from about 3% to about 5%, from about 8% to about 12% or from about 10% to about 12%. Non-limiting examples of cationic surfactants include alkyldimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, ester quats, and combinations thereof.

When included therein the detergent will usually contain from about 0.2% to about 40% by weight of a nonionic surfactant, for example from about 0.5% to about 30%, in particular from about 1% to about 20%, from about 3% to about 10%, such as from about 3% to about 5%, from about 8% to about 12%, or from about 10% to about 12%. Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof. When included therein the detergent will usually contain from about 0.01%

to about 10 % by weight of a semipolar surfactant. Non-limiting examples of semipolar surfactants include amine oxides (AO) such as alkyldimethylamineoxide, N-(coco alkyl)-N,N-dimethylamine oxide and N-(tallow-alkyl)-N,N-bis(2-hydroxyethyl)amine oxide, and combinations thereof. When included therein the detergent will usually contain from about 0.01% to about 10 % by weight of a zwitterionic surfactant. Non-limiting examples of zwitterionic surfactants include betaines such as alkyldimethylbetaines, sulfobetaines, and combinations thereof.

Builders and Co-Builders

The detergent composition may contain about 0-65% by weight, such as about 5% to about 50%, such as from about 0.5 to about 20% of a detergent builder or co-builder, or a mixture thereof. In a dish wash detergent, the level of builder is typically 40-65%, particularly 50-65%. The builder and/or co-builder may particularly be a chelating agent that forms water-soluble complexes with Ca and Mg. Any builder and/or co-builder known in the art for use in cleaning detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as 2,2'-iminodiethan-1-ol), triethanolamine (TEA, also known as 2,2',2"-nitrilotriethan-1-ol), and (carboxymethyl)inulin (CMI), and combinations thereof.

The detergent composition may also contain 0-50% by weight, such as about 5% to about 30%, of a detergent co-builder. The detergent composition may include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-N,N'-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), N-(2-hydroxyethyl)iminodiacetic acid (EDG), aspartic acid-N-monoacetic acid (ASMA), aspartic acid-N,N-diacetic acid (ASDA), aspartic acid-N-monopropionic acid (ASMP), iminodisuccinic acid (IDA), N-(2-sulfomethyl)-aspartic acid (SMAS), N-(2-sulfoethyl)-aspartic acid (SEAS), N-(2-sulfomethyl)-glutamic acid (SMGL), N-(2-sulfoethyl)-glutamic acid (SEGL), N-methyliminodiacetic acid (MIDA), α-alanine-N,N-diacetic acid (α-ALDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA) , taurine-N,N-diacetic acid (TUDA) and sulfomethyl-N,N-diacetic acid (SMDA), N-(2-hydroxyethyl)ethylenediamine-N,N',N"-triacetic acid (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053

Bleaching Systems

The detergent may contain 0-30% by weight, such as about 1% to about 20%, such as from about 0.01 to about 10 wt% of a bleaching system. Any bleaching system comprising components known in the art for use in cleaning detergents may be utilized. Suitable bleaching system components include sources of hydrogen peroxide; sources of peracids; and bleach catalysts or boosters.

Sources of hydrogen peroxide:
Suitable sources of hydrogen peroxide are inorganic persalts, including alkali metal salts such as sodium percarbonate and sodium perborates (usually mono- or tetrahydrate), and hydrogen peroxide-urea (1/1).

Sources of peracids:
Peracids may be (a) incorporated directly as preformed peracids or (b) formed in situ in the wash liquor from hydrogen peroxide and a bleach activator (perhydrolysis) or (c) formed in situ in the wash liquor from hydrogen peroxide and a perhydrolase and a suitable substrate for the latter, e.g., an ester.

a) Suitable preformed peracids include, but are not limited to, peroxycarboxylic acids such as peroxybenzoic acid and its ring-substituted derivatives, peroxy-α-naphthoic acid, peroxyphthalic acid, peroxylauric acid, peroxystearic acid, ε-phthalimidoperoxycaproic acid [phthalimidoperoxyhexanoic acid (PAP)], and o-carboxybenzamidoperoxycaproic acid; aliphatic and aromatic diperoxydicarboxylic acids such as diperoxydodecanedioic acid, diperoxyazelaic acid, diperoxysebacic acid, diperoxybrassylic acid, 2-decyldiperoxybutanedioic acid, and diperoxyphthalic, -isophthalic and -terephthalic acids; perimidic acids; peroxymonosulfuric acid; peroxydisulfuric acid; peroxyphosphoric acid; peroxysilicic acid; and mixtures of said compounds. It is understood that the peracids mentioned may in some cases

be best added as suitable salts, such as alkali metal salts (e.g., Oxone®) or alkaline earth-metal salts.

b) Suitable bleach activators include those belonging to the class of esters, amides, imides, nitriles or anhydrides and, where applicable, salts thereof. Suitable examples are tetraacetylethylenediamine (TAED), sodium 4-[(3,5,5-trimethylhexanoyl)oxy]benzene-1-sulfonate (ISONOBS), sodium 4-(dodecanoyloxy)benzene-1-sulfonate (LOBS), sodium 4-(decanoyloxy)benzene-1-sulfonate, 4-(decanoyloxy)benzoic acid (DOBA), sodium 4-(nonanoyloxy)benzene-1-sulfonate (NOBS), and/or those disclosed in WO98/17767. A particular family of bleach activators of interest was disclosed in EP624154 and particularly preferred in that family is acetyl triethyl citrate (ATC). ATC or a short chain triglyceride like triacetin has the advantage that they are environmentally friendly. Furthermore, acetyl triethyl citrate and triacetin have good hydrolytical stability in the product upon storage and are efficient bleach activators. Finally, ATC is multifunctional, as the citrate released in the perhydrolysis reaction may function as a builder.

Bleach catalysts and boosters

The bleaching system may also include a bleach catalyst or booster.

Some non-limiting examples of bleach catalysts that may be used in the compositions of the present invention include manganese oxalate, manganese acetate, manganese-collagen, cobalt-amine catalysts and manganese triazacyclononane (MnTACN) catalysts; particularly preferred are complexes of manganese with 1,4,7-trimethyl-1,4,7-triazacyclononane (Me3-TACN) or 1,2,4,7-tetramethyl-1,4,7-triazacyclononane (Me4-TACN), in particular Me3-TACN, such as the dinuclear manganese complex [(Me3-TACN)Mn(O)3Mn(Me3-TACN)](PF6)2, and [2,2',2''-nitrilotris(ethane-1,2-diylazanylylidene-κN-methanylylidene)triphenolato-κ3O]manganese(III). The bleach catalysts may also be other metal compounds; such as iron or cobalt complexes.

In some embodiments, where a source of a peracid is included, an organic bleach catalyst or bleach booster may be used having one of the following formulae:

(i)

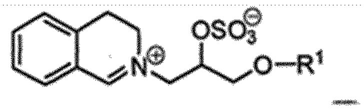

(ii)

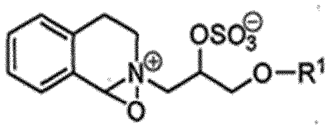

(iii) and mixtures thereof; wherein each R1 is independently a branched alkyl group containing from 9 to 24 carbons or linear alkyl group containing from 11 to 24 carbons, preferably each R1 is independently a branched alkyl group containing from 9 to 18 carbons or linear alkyl group containing from 11 to 18 carbons, more preferably each R1 is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl.

Other exemplary bleaching systems are described, e.g. in WO2007/087258, WO2007/087244, WO2007/087259, EP1867708 (Vitamin K) and WO2007/087242. Suitable photobleaches may for example be sulfonated zinc or aluminium phthalocyanines.

Metal care agents

Metal care agents may prevent or reduce the tarnishing, corrosion or oxidation of metals, including aluminium, stainless steel and non-ferrous metals, such as silver and copper. Suitable examples include one or more of the following:

(a) benzatriazoles, including benzotriazole or bis-benzotriazole and substituted derivatives thereof. Benzotriazole derivatives are those compounds in which the available substitution sites on the aromatic ring are partially or completely substituted. Suitable substituents include linear or branch-chain Ci-C20- alkyl groups (e.g., C1-C20- alkyl groups) and hydroxyl, thio, phenyl or halogen such as fluorine, chlorine, bromine and iodine.

(b) metal salts and complexes chosen from the group consisting of zinc, manganese, titanium, zirconium, hafnium,

vanadium, cobalt, gallium and cerium salts and/or complexes, the metals being in one of the oxidation states II, III, IV, V or VI. In one aspect, suitable metal salts and/or metal complexes may be chosen from the group consisting of Mn(II) sulphate, Mn(II) citrate, Mn(II) stearate, Mn(II) acetylacetonate, K^TiF6 (e.g., K2TiF6), K^ZrF6 (e.g., K2ZrF6), CoSO4, Co(NOs)2 and Ce(NOs)3, zinc salts, for example zinc sulphate, hydrozincite or zinc acetate.;

(c) silicates, including sodium or potassium silicate, sodium disilicate, sodium metasilicate, crystalline phyllosilicate and mixtures thereof.

Further suitable organic and inorganic redox-active substances that act as silver/copper corrosion inhibitors are disclosed in WO 94/26860 and WO 94/26859. Preferably the composition of the invention comprises from 0.1 to 5% by weight of the composition of a metal care agent, preferably the metal care agent is a zinc salt.

Hydrotropes

The detergent may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Suitable examples include PVP-K15, PVP-K30, ChromaBond S-400, ChromaBond S- 403E and Chromabond S-100 from Ashland Aqualon, and Sokalan® HP 165, Sokalan® HP 50 (Dispersing agent), Sokalan® HP 53 (Dispersing agent), Sokalan® HP 59 (Dispersing agent), Sokalan® HP 56 (dye transfer inhibitor), Sokalan® HP 66 K (dye transfer inhibitor) from BASF. Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated. Particularly preferred polymer is ethoxylated homopolymer Sokalan® HP 20 from BASF.

Fabric hueing agents

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO2005/03274, WO2005/03275, WO2005/03276 and EP1876226 (hereby incorporated by reference). The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO2007/087243.

Enzymes

The detergent additive as well as the detergent composition may comprise one or more additional enzymes such as one or more proteases lipase, cutinase, pectinase, arabinase, galactanase, xylanase, oxidase, e.g., a laccase, and/or peroxidase.

In general, the properties of the selected enzyme(s) should be compatible with the selected detergent, (i.e., pH-optimum, compatibility with other enzymatic and non-enzymatic ingredients, etc.), and the enzyme(s) should be present in effective amounts.

**Proteases**

Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families. The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family. Examples of subtilases are those derived from *Bacillus* such as *Bacillus lentus, Bacillus alkalophilus, Bacillus subtilis, Bacillus amyloliquefaciens, Bacillus pumilus* and *Bacillus gibsonii* described in; US7262042 and WO09/021867, and *Subtilisin lentus, Subtilisin* Novo, *subtilisin* Carlsberg, *Bacillus licheniformis, subtilisin* BPN', *subtilisin* 309, *subtilisin* 147 and *subtilisin* 168 and e.g. protease PD138 described in (WO93/18140). Other useful proteases may be those described in WO01/016285 and WO002/016547. Examples of trypsin-like proteases are trypsin (e.g. of porcine or bovine origin) and the *Fusarium* protease described in WO94/25583 and WO05/040372, and the chymotrypsin proteases derived from *Cellumonas* described in WO05/052161 and WO05/052146. A further preferred protease is the alkaline protease from *Bacillus lentus* DSM 5483, as described for example in WO95/23221, and variants thereof which are described in WO92/21760, WO95/23221, EP1921147 and EP1921148. Examples of metalloproteases are the neutral metalloprotease as described in WO07/044993 (Proctor & Gamble/Genencor Int.) such as those derived from *Bacillus amyloliquefaciens.* Examples of useful proteases are the variants described in: WO89/06279 WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 24, 27, 42, 55, 59, 60, 66, 74, 85, 96, 97, 98, 99, 100, 101, 102, 104, 116, 118, 121, 126, 127, 128, 154, 156, 157, 158, 161, 164, 176, 179, 182, 185, 188, 189, 193, 198, 199, 200, 203, 206, 211, 212, 216, 218, 226, 229, 230, 239, 246, 255, 256, 268 and 269 wherein the positions correspond to the positions of the *Bacillus lentus* protease shown in SEQ ID NO: 1 of WO 2016/001449. More preferred the protease variants may comprise one or more of the mutations selected from the group consisting of: S3T, V4I, S9R, S9E, A15T, S24G, S24R, K27R, N42R, S55P, G59E, G59D, N60D, N60E, V66A, N74D, S85R, A96S, S97G, S97D, S97A, S97SD, S99E, S99D, S99G, S99M, S99N, S99R, S99H, S101A, V102I, V102Y, V102N, S104A, G116V, G116R, H118D, H118N, A120S, S126L, P127Q, S128A, S154D, A156E, G157D, G157P, S158E, Y161A, R164S, Q176E, N179E, S182E, Q185N, A188P, G189E, V193M, N198D, V199I, Y203W, S206G, L211Q, L211D, N212D, N212S, M216S, A226V, K229L, Q230H, Q239R, N246K, N255W, N255D, N255E, L256E, L256D T268A and R269H. The protease variants are preferably variants of the *Bacillus lentus* protease shown in SEQ ID NO: 1 of WO2016/001449, the *Bacillus amylolichenifaciens* protease (BPN') shown in SEQ ID NO: 2 of WO2016/001449. The protease variants preferably have at least 80% sequence identity to SEQ ID NO: 1 or SEQ ID NO: 2 of WO 2016/001449. A protease variant comprising a substitution at one or more positions corresponding to positions 171, 173, 175, 179, or 180 of SEQ ID NO: 1 of WO2004/067737, wherein said protease variant has a sequence identity of at least 75% but less than 100% to SEQ ID NO: 1 of WO2004/067737. Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Blaze®, Blaze Evity® 100T, Blaze Evity® 125T, Blaze Evity® 150T, Neutrase®, Everlase® and Esperase® (Novozymes A/S), those sold under the tradename Maxatase®, Maxacal®, Maxapem®, Purafect Ox®, Purafect OxP®, Puramax®, FN2®, FN3®, FN4®, Excellase®, Excellenz P1000™, Excellenz P1250™, Eraser®, Preferenz P100™, Purafect Prime®, Preferenz P110™, Effectenz P1000™, Purafect®™, Effectenz P1050™, Purafect Ox®™, Effectenz P2000™, Purafast®, Properase®, Opticlean® and Optimase® (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

**Lipases and Cutinases:**

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP258068 and EP305216, cutinase from *Humicola,* e.g. H. insolens (WO96/13580), lipase from strains of *Pseudomonas* (some of these now renamed to *Burkholderia),* e.g. P. alcaligenes or P. pseudoalcaligenes (EP218272), P. cepacia (EP331376), P. sp. strain SD705 (WO95/06720 & WO96/27002), *P. wisconsinensis* (WO96/12012), GDSL-type *Streptomyces* lipases (WO10/065455), cutinase from Magnaporthe grisea (WO10/107560), cutinase from Pseudomonas mendocina (US5,389,536), lipase from *Thermobifida fusca* (WO11/084412), *GeoBacillus stearothermophilus* lipase (WO11/084417), lipase from *Bacillus subtilis* (WO11/084599), and lipase from *Streptomyces griseus* (WO11/150157) and *S. pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include Lipolase™, Lipex™; Lipolex™ and Lipoclean™ (Novozymes A/S), Lumafast (originally from Genencor) and Lipomax (originally from Gist-Brocades).

Still other examples are lipases sometimes referred to as acyltransferases or perhydrolases, e.g. acyltransferases with homology to Candida antarctica lipase A (WO10/111143), acyltransferase from *Mycobacterium smegmatis* (WO05/56782), perhydrolases from the CE 7 family (WO09/67279), and variants of the M. smegmatis perhydrolase in particular the S54V variant used in the commercial product Gentle Power Bleach from Huntsman Textile Effects Pte Ltd (WO10/100028).

**Peroxidases/Oxidases**

Suitable peroxidases/oxidases include those of plant, bacterial or fungal origin. Chemically modified or protein engineered mutants are included. Examples of useful peroxidases include peroxidases from *Coprinus,* e.g., from *C. cinereus,* and variants thereof as those described in WO 93/24618, WO 95/10602, and WO 98/15257. Commercially available peroxidases include Guardzyme™ (Novozymes A/S). A peroxidase is a peroxidase enzyme comprised by the enzyme classification EC 1.11.1.7, as set out by the Nomenclature Committee of the International Union of Biochemistry and Molecular Biology (IUBMB), or any fragment derived therefrom, exhibiting peroxidase activity. A suitable peroxidase includes a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions. Preferably, the haloperoxidase is a vanadium haloperoxidase, i.e., a vanadate-containing haloperoxidase. Haloperoxidases have been isolated from many different fungi, in particular from the fungus group dematiaceous hyphomycetes, such as *Caldariomyces,* e.g., *C. fumago, Alternaria, Curvularia,* e.g., *C. verruculosa* and *C. inaequalis, Drechslera, Ulocladium* and *Botrytis.*

Haloperoxidases have also been isolated from bacteria such as *Pseudomonas,* e.g., *P*. pyrrocinia and Streptomyces, e.g., S. aureofaciens.

A suitable oxidase includes in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5). Preferred laccase enzymes are enzymes of microbial origin. The enzymes may be derived from plants, bacteria or fungi (including filamentous fungi and yeasts). Suitable examples from fungi include a laccase derivable from a strain of *Aspergillus, Neurospora,* e.g., *N. crassa, Podospora, Botrytis, Collybia, Fomes, Lentinus, Pleurotus, Trametes,* e.g., *T. villosa* and *T. versicolor, Rhizoctonia,* e.g., R. *solani, Coprinopsis,* e.g., *C. cinerea, C. comatus, C. friesii, and C. plicatilis, Psathyrella,* e.g., P. *condelleana, Panaeolus,* e.g., P. *papilionaceus, Myceliophthora,* e.g., *M. thermophila, Schytalidium,* e.g., *S. thermophilum, Polyporus,* e.g., *P. pinsitus, Phlebia,* e.g., *P. radiata* (WO 92/01046), or *Coriolus,* e.g., *C. hirsutus* (JP 2238885). Suitable examples from bacteria include a laccase derivable from a strain of *Bacillus.* A laccase derived from *Coprinopsis* or *Myceliophthora* is preferred; in particular, a laccase derived from *Coprinopsis cinerea,* as disclosed in WO 97/08325; or from *Myceliophthora thermophila,* as disclosed in WO 95/33836.

<u>Dispersants</u>

The cleaning compositions of the present invention can also contain dispersants. In particular, powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

Dye Transfer Inhibiting Agents

The cleaning compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

Fluorescent whitening agent

The cleaning compositions of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(N-methyl-N-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2H-naphtho[1,2-d][1,2,3]triazol-2-yl)-2-[(E)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins. Suitable fluorescent brightener levels include lower levels of from about 0.01, from 0.05, from about 0.1 or even from about 0.2 wt % to upper levels of 0.5 or even 0.75 wt%.

Soil release polymers

The cleaning compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers is amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore, random graft co-polymers are suitable soil release polymers. Suitable graft co-polymers are described in more detail in WO 2007/138054, WO 2006/108856 and WO 2006/113314 (hereby incorporated by reference). Suitable polyethylene glycol polymers include random graft co-polymers comprising: (i) hydrophilic backbone comprising polyethylene glycol; and (ii) side chain(s) selected from the group consisting of: C4-C25 alkyl group, polypropylene, polybutylene, vinyl ester of a saturated C1-C6 mono-carboxylic acid, CI-C 6 alkyl ester of acrylic or methacrylic acid, and mixtures thereof. Suitable polyethylene glycol polymers have a polyethylene glycol backbone with random grafted polyvinyl acetate side chains. The average molecular weight of the polyethylene glycol backbone can be in the range of from 2,000 Da to 20,000 Da, or from 4,000 Da to 8,000 Da. The molecular weight ratio of the polyethylene glycol backbone to the polyvinyl acetate side chains can be in the range of from 1: 1 to 1:5, or from 1: 1.2 to 1:2. The average number of graft sites per ethylene oxide units can be less than 1, or less than 0.8, the average number of graft sites per ethylene oxide units can be in the range of from 0.5 to 0.9, or the average number of graft sites per ethylene oxide units can be in the range of from 0.1 to 0.5, or from 0.2 to 0.4. A suitable polyethylene glycol polymer is Sokalan HP22. Other soil release polymers are substituted polysaccharide structures especially substituted cellulosic structures such as modified cellulose deriviatives such as those described in EP 1867808 or WO 2003/040279 (both are hereby incorporated by reference). Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents

The cleaning compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose-based polymers described under soil release polymers above may also function as anti-redeposition agents.

Rheology Modifiers

The cleaning compositions of the present invention may also include one or more rheology modifiers, structurants or thickeners, as distinct from viscosity reducing agents. The rheology modifiers are selected from the group consisting of non-polymeric crystalline, hydroxyfunctional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable cleaning composition components include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

Formulation of detergent products

The cleaning composition of the invention may be in any convenient form, e.g., a bar, a homogenous tablet, a tablet having two or more layers, a pouch having one or more compartments, a regular or compact powder, a granule, a paste, a gel, or a regular, compact or concentrated liquid.

Pouches can be configured as single or multicompartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water-soluble film. The compartment for liquid components can be different in composition than compartments containing solids: US2009/0011970 A1.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent. A liquid or gel detergent may be non-aqueous.

Granular detergent formulations

Non-dusting granulates may be produced, e.g. as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic

acid or boric acid according to established methods. Protected enzymes may be prepared according to the method disclosed in EP 238,216.

The dispersin may be formulated as a granule for example as a co-granule that combines one or more enzymes. Each enzyme will then be present in more granules securing a more uniform distribution of enzymes in the detergent. This also reduces the physical segregation of different enzymes due to different particle sizes. Methods for producing multi-enzyme co-granulate for the detergent industry is disclosed in the IP.com disclosure IPCOM000200739D.

Another example of formulation of enzymes by the use of co-granulates are disclosed in WO 2013/188331, which relates to a detergent composition comprising (a) a multi-enzyme co-granule; (b) less than 10 wt zeolite (anhydrous basis); and (c) less than 10 wt phosphate salt (anhydrous basis), wherein said enzyme co-granule comprises from 10 to 98 wt% moisture sink component and the composition additionally comprises from 20 to 80 wt% detergent moisture sink component. WO 2013/188331 also relates to a method of treating and/or cleaning a surface, preferably a fabric surface comprising the steps of (i) contacting said surface with the detergent composition as claimed and described herein in aqueous wash liquor, (ii) rinsing and/or drying the surface.

An embodiment of the invention relates to an enzyme granule/particle comprising the dispersin and at least one carbohydrase and a cleaning component, wherein the dispersin is a polypeptide having hexosaminidase activity and at least 60% sequence identity to the polypeptide of SEQ ID NO: 17, and wherein the carbohydrase is an alpha-amylase. The granule is composed of a core, and optionally one or more coatings (outer layers) surrounding the core. Typically, the granule/particle size, measured as equivalent spherical diameter (volume based average particle size), of the granule is 20-2000 pm, particularly 50-1500 pm, 100-1500 pm or 250-1200 pm. The core may include additional materials such as fillers, fibre materials (cellulose or synthetic fibres), stabilizing agents, solubilising agents, suspension agents, viscosity regulating agents, light spheres, plasticizers, salts, lubricants and fragrances. The core may include binders, such as synthetic polymer, wax, fat, or carbohydrate. The core may comprise a salt of a multivalent cation, a reducing agent, an antioxidant, a peroxide decomposing catalyst and/or an acidic buffer component, typically as a homogenous blend. The core may consist of an inert particle with the enzyme absorbed into it, or applied onto the surface, e.g., by fluid bed coating. The core may have a diameter of 20-2000 μm, particularly 50-1500 μm, 100-1500 μm or 250-1200 μm. The core can be prepared by granulating a blend of the ingredients, e.g., by a method comprising granulation techniques such as crystallization, precipitation, pan-coating, fluid bed coating, fluid bed agglomeration, rotary atomization, extrusion, prilling, spheronization, size reduction methods, drum granulation, and/or high shear granulation.

Methods for preparing the core can be found in Handbook of Powder Technology; Particle size enlargement by C. E. Capes; Volume 1; 1980; Elsevier.

The core of the enzyme granule/particle may be surrounded by at least one coating, e.g., to improve the storage stability, to reduce dust formation during handling, or for coloring the granule. The optional coating(s) may include a salt coating, or other suitable coating materials, such as polyethylene glycol (PEG), methyl hydroxy-propyl cellulose (MHPC) and polyvinyl alcohol (PVA). Examples of enzyme granules with multiple coatings are shown in WO 93/07263 and WO 97/23606. The coating may be applied in an amount of at least 0.1% by weight of the core, e.g., at least 0.5%, 1% or 5%. The amount may be at most 100%, 70%, 50%, 40% or 30%. The coating is preferably at least 0.1 μm thick, particularly at least 0.5 μm, at least 1 μm or at least 5 μm. In a one embodiment, the thickness of the coating is below 100 μm. In another embodiment, the thickness of the coating is below 60 μm. In an even more particular embodiment the total thickness of the coating is below 40 μm. The coating should encapsulate the core unit by forming a substantially continuous layer. A substantially continuous layer is to be understood as a coating having few or no holes, so that the core unit it is encapsulating/enclosing has few or none uncoated areas. The layer or coating should be homogeneous in thickness. The coating can further contain other materials as known in the art, e.g., fillers, antisticking agents, pigments, dyes, plasticizers and/or binders, such as titanium dioxide, kaolin, calcium carbonate or talc. A salt coating may comprise at least 60% by weight w/w of a salt, e.g., at least 65%, at least 70%, at least 75%, at least 80%, at least 85%, at least 90%, at least 95% or at least 99% by weight w/w. The salt may be added from a salt solution where the salt is completely dissolved or from a salt suspension wherein the fine particles is less than 50 μm, such as less than 10 μm or less than 5 μm. The salt coating may comprise a single salt or a mixture of two or more salts. The salt may be water soluble and may have a solubility at least 0.1 grams in 100 g of water at 20°C, preferably at least 0.5 g per 100 g water, *e.g.,* at least 1 g per 100 g water, *e.g.,* at least 5 g per 100 g water. The salt may be an inorganic salt, *e.g.*, salts of sulfate, sulfite, phosphate, phosphonate, nitrate, chloride or carbonate or salts of simple organic acids (less than 10 carbon atoms, *e.g.*, 6 or less carbon atoms) such as citrate, malonate or acetate. Examples of cations in these salts are alkali or earth alkali metal ions, the ammonium ion or metal ions of the first transition series, such as sodium, potassium, magnesium, calcium, zinc or aluminium. Examples of anions include chloride, bromide, iodide, sulfate, sulfite, bisulfite, thiosulfate, phosphate, monobasic phosphate, dibasic phosphate, hypophosphite, dihydrogen pyrophosphate, tetraborate, borate, carbonate, bicarbonate, metasilicate, citrate, malate, maleate, malonate, succinate, lactate, formate, acetate, butyrate, propionate, benzoate, tartrate, ascorbate or gluconate. In particular alkali- or earth alkali metal salts of sulfate, sulfite, phosphate, phosphonate, nitrate, chloride or carbonate or salts of simple organic acids such as citrate, malonate or acetate may be used. The salt in the coating may have a constant humidity at 20°C above 60%, particularly above 70%, above 80% or above 85%, or it may be another

hydrate form of such a salt (e.g., anhydrate). The salt coating may be as described in WO 00/01793 or WO 2006/034710. Specific examples of suitable salts are NaCl ($CH_{20°C}$=76%), $Na_2CO_3$ ($CH_{20°C}$=92%), $NaNO_3$ ($CH_{20°C}$=73%), $Na_2HPO_4$ ($CH_{20°C}$=95%), $Na_3PO_4$ ($CH_{25°C}$=92%), $NH_4Cl$ ($CH_{20°C}$=79.5%), $(NH_4)_2HPO_4$ ($CH_{20°C}$=93,0%), $NH_4H_2PO_4$ ($CH_{20°C}$=93.1%), $(NH_4)_2SO_4$ ($CH_{20°C}$=81.1%), KCl ($CH_{20°C}$=85%), $K_2HPO_4$ ($CH_{20°C}$=92%), $KH_2PO_4$ ($CH_{20°C}$=96.5%), $KNO_3$ ($CH_{20°C}$=93.5%), $Na_2SO_4$ ($CH_{20°C}$=93%), $K_2SO_4$ ($CH_{20°C}$=98%), $KH_3O_4$ ($CH_{20°C}$=86%), $MgSO_4$ ($CH_{20°C}$=90%), $Zn_3O_4$ ($CH_{20°C}$=90%) and sodium citrate ($CH_{25°C}$=86%). Other examples include $NaH_2PO_4$, $(NH_4)H_2PO_4$, $CuSO_4$, $Mg(NO_3)_2$ and magnesium acetate. The salt may be in anhydrous form, or it may be a hydrated salt, i.e. a crystalline salt hydrate with bound water(s) of crystallization, such as described in WO 99/32595. Specific examples include anhydrous sodium sulfate ($Na_2SO_4$), anhydrous magnesium sulfate ($MgSO_4$), magnesium sulfate heptahydrate ($MgSO_4$ $7H_2O$), zinc sulfate heptahydrate ($ZnSO_4$ $7H_2O$), sodium phosphate dibasic heptahydrate ($Na_2HPO_4$ $7H_2O$), magnesium nitrate hexahydrate ($Mg(NO_3)_2$ ($6H_2O$)), sodium citrate dihydrate and magnesium acetate tetrahydrate. Preferably the salt is applied as a solution of the salt, e.g., using a fluid bed.

One embodiment of the present invention provides a granule, which comprises:

(a) a core comprising a dispersin and a carbohydrase, wherein the dispersin is a polypeptide having hexosaminidase activity and at least 60% sequence identity to the polypeptide of SEQ ID NO: 17, and wherein the carbohydrase is an alpha-amylase, and
(b) optionally a coating consisting of one or more layer(s) surrounding the core.

One embodiment of the invention relates to a granule, which comprises:

(a) a core comprising a dispersin and an alpha-amylase wherein the alpha-amylase is selected from the group consisting of a polypeptide having at least 60%, at least 65%, at least 70%, at least 75%, at least 80%, at least 85%, at least 90%, at least 95%, at least 98% or 100% sequence identity to the polypeptide shown in SEQ ID NO: 30, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 31 , a polypeptide having at least 60%, at least 65%, at least 70%, at least 75%, at least 80%, at least 85%, at least 90%, at least 95%, at least 98% or 100% sequence identity to the polypeptide shown in SEQ ID NO: 32, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 33, and a polypeptide having at least 60%, at least 65%, at least 70%, at least 75%, at least 80%, at least 85%, at least 90%, at least 95%, at least 98% or 100% sequence identity to the polypeptide shown in SEQ ID NO: 43 and wherein the dispersin has at least 60%, at least 65%, at least 70%, at least 75%, at least 80%, at least 85%, at least 90%, at least 95%, at least 98% or 100% sequence identity to the amino acid sequence shown in SEQ ID NO: 17, and
(b) optionally a coating consisting of one or more layer(s) surrounding the core.

**Uses**

The present invention is also directed to methods and use of the compositions of the invention in e.g. laundry/textile/fabric (House hold laundry washing, Industrial laundry washing) and hard surface cleaning (ADW, car wash, Industrial surface). The compositions of the invention comprise a blend of dispersin and carbohydrase, wherein the dispersin is a polypeptide having hexosaminidase activity and at least 60% sequence identity to the polypeptide of SEQ ID NO: 17, and wherein the carbohydrase is an alpha-amylase, which effectively reduce or remove organic components, such as biofilm and components hereof e.g. mannan PNAG from surfaces such as textiles and hard surfaces e.g. dishes.

The compositions of the invention comprise a blend of dispersin and carbohydrase, wherein the dispersin is a polypeptide having hexosaminidase activity and at least 60% sequence identity to the polypeptide of SEQ ID NO: 17, and wherein the carbohydrase is an alpha-amylase, and effectively reduce or remove organic components, such as mannan, starch, cellulose, xyloglucan, biofilm and components hereof e.g. PNAG from surfaces such as textiles and hard surfaces e.g. dishes. One embodiment of the invention relates to the use of a cleaning composition comprising a dispersin, a carbohydrase, selected from an alpha-amylase, and at least one cleaning component for reduction or removal of stains such as biofilm and components hereof e.g. PNAG and at least one carbohydrase and a cleaning component, wherein the dispersin is a polypeptide having hexosaminidase activity and at least 60% sequence identity to the polypeptide of SEQ ID NO: 17, and wherein the carbohydrase is an amylase, of an item, wherein the item is a textile or a hard surface.

One embodiment of the invention relates to the use of a cleaning composition comprising a dispersin, at least one carbohydrase and a cleaning component, wherein the dispersin is a polypeptide having hexosaminidase activity and at least 60% sequence identity to the polypeptide of SEQ ID NO: 17, and wherein the carbohydrase is an alpha-amylase for deep cleaning of an item, wherein the item is a textile or a surface.

One embodiment of the invention relates to the use of a composition comprising a dispersin and a carbohydrase,

wherein the dispersin is a polypeptide having hexosaminidase activity and at least 60% sequence identity to the polypeptide of SEQ ID NO: 17, and wherein the carbohydrase is an alpha-amylase for reduction or removal of polysaccharide stains and/or compounds such as mannan, starch, cellulose, xyloglucan, biofilm and components hereof e.g. PNAG of an item. One embodiment of the invention relates to the use of a cleaning composition comprising a dispersin and a carbohydrase, wherein the dispersin is a polypeptide having hexosaminidase activity and at least 60% sequence identity to the polypeptide of SEQ ID NO: 17, and wherein the carbohydrase is an alpha-amylase for reduction or removal of polysaccharide stains and/or compounds such as mannan, starch, cellulose, xyloglucan and PNAG of an item such as textile. One embodiment of the invention relates to the use of a cleaning composition comprising a dispersin and a carbohydrase, wherein the dispersin is a polypeptide having hexosaminidase activity and at least 60% sequence identity to the polypeptide of SEQ ID NO: 17, and wherein the carbohydrase is an alpha-amylase for deep cleaning when the cleaning composition is applied in e.g. laundry process.

One embodiment of the invention relates to the use of a composition comprising a dispersin and carbohydrase, selected from a an alpha-amylase for reduction of redeposition or reduction of malodor, wherein the dispersin is a polypeptide having hexosaminidase activity and at least 60% sequence identity to the polypeptide of SEQ ID NO: 17.

One embodiment of the invention relates to the use of a composition comprising a dispersin and an alpha-amylase for reduction of malodor, wherein the dispersin is a polypeptide having hexosaminidase activity and at least 60% sequence identity to the polypeptide of SEQ ID NO: 17.

One embodiment of the invention relates to the use of a cleaning composition comprising a dispersin and carbohydrase, selected from an alpha-amylase for reduction of redeposition or reduction of malodor when the cleaning composition is applied in e.g. laundry process, wherein the dispersin is a polypeptide having hexosaminidase activity and at least 60% sequence identity to the polypeptide of SEQ ID NO: 17. One embodiment of the invention relates to the use of a cleaning composition comprising a dispersin and carbohydrase, selected from an alpha-amylase for reduction of redeposition or reduction of malodor on an item e.g. textile, wherein the dispersin is a polypeptide having hexosaminidase activity and at least 60% sequence identity to the polypeptide of SEQ ID NO: 17. In one embodiment, the composition is an anti-redeposition composition.

One embodiment of the invention relates to the use of a cleaning composition comprising a dispersin and an alpha-amylase for deep cleaning of an item or reduction of redeposition or malodor, wherein the dispersin is a polypeptide having hexosaminidase activity and at least 60% sequence identity to the polypeptide of SEQ ID NO: 17, and wherein the amylase selected from a polypeptide having at least 60%, at least 65%, at least 70%, at least 75%, at least 80%, at least 85%, at least 90%, at least 95%, at least 98% or 100% sequence identity to the polypeptide shown in SEQ ID NO: 30, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 31 , a polypeptide having at least 60%, at least 65%, at least 70%, at least 75%, at least 80%, at least 85%, at least 90%, at least 95%, at least 98% or 100% sequence identity to the polypeptide shown in SEQ ID NO: 32, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 33 and a polypeptide having at least 60%, at least 65%, at least 70%, at least 75%, at least 80%, at least 85%, at least 90%, at least 95%, at least 98% or 100% sequence identity to the polypeptide shown in SEQ ID NO: 43.

One embodiment of the invention relates to the use of a cleaning composition comprising a dispersin and alpha-amylase for deep cleaning of an item or reduction of redeposition or malodor, wherein the alpha-amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 30, and wherein the dispersin is selected from: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17.

One embodiment of the invention relates to the use of a cleaning composition comprising a dispersin and alpha-amylase for deep cleaning of an item or reduction of redeposition or malodor, wherein the alpha-amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 31, and wherein the dispersin is selected from: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17.

One embodiment of the invention relates to the use of a cleaning composition comprising a dispersin and alpha-amylase for deep cleaning of an item or reduction of redeposition or malodor, wherein the alpha-amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 32, and wherein the dispersin is selected from: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17.

One embodiment of the invention relates to the use of a cleaning composition comprising a dispersin and alpha-amylase for deep cleaning of an item or reduction of redeposition or malodor, wherein the alpha-amylase is a polypeptide

having at least 60%, at least 65%, at least 70%, at least 75%, at least 80%, at least 85%, at least 90%, at least 95%, at least 98% or 100% sequence identity to the polypeptide shown in SEQ ID NO: 33, and wherein the dispersin is selected from: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17.

One embodiment of the invention relates to the use of a cleaning composition comprising a dispersin and alpha-amylase for deep cleaning of an item or reduction of redeposition or malodor, wherein the alpha-amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 43, and wherein the dispersin is selected from: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17

The invention further relates to a method of deep cleaning of an item, wherein the item may be textile or hard surface preferably is a textile,

One embodiment of the invention relates to a method of deep cleaning on an item, comprising the steps of:

a) contacting the item with a cleaning composition according to the invention; and
b) and optionally rinsing the item, wherein the item is preferably a textile.

One embodiment of the invention relates to a method of deep cleaning on an item, comprising the steps of:

a) contacting the item with a solution comprising an enzyme mixture comprising a dispersin, wherein the dispersin is a polypeptide having hexosaminidase activity and at least 60% sequence identity to the polypeptide of SEQ ID NO: 17, a carbohydrase, wherein the carbohydrase is an alpha-amylase, and a cleaning component, wherein the cleaning component is selected from 0.1 to 50 wt% of at least one a surfactant; 0.5 to 30 wt% of at least one builder; and 0.01 to 20 wt% of at least one bleach component; and
b) and optionally rinsing the item, wherein the item is preferably a textile.

One embodiment of the invention relates to a method of deep cleaning on an item, comprising the steps of:

a) contacting the item with a solution comprising an enzyme mixture comprising a dispersin, wherein the dispersin is a polypeptide having hexosaminidase activity and at least 60% sequence identity to the polypeptide of SEQ ID NO: 17, an alpha-amylase; and a cleaning component, wherein the cleaning component is selected from 0.1 to 50 wt% of at least one a surfactant; 0.5 to 30 wt% of at least one builder; and 0.01 to 20 wt% of at least one bleach component; and
b) optionally rinsing the item, wherein the item is preferably a textile and

wherein the alpha-amylase is selected from the group consisting of a polypeptide having at least 60%, at least 65%, at least 70%, at least 75%, at least 80%, at least 85%, at least 90%, at least 95%, at least 98% or 100% sequence identity to the polypeptide shown in SEQ ID NO: 30, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 31, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 32, a polypeptide having at least 60%, at least 65%, at least 70%, at least 75%, at least 80%, at least 85%, at least 90%, at least 95%, at least 98% or 100% sequence identity to the polypeptide shown in SEQ ID NO: 33, and a polypeptide having at least 60%, at least 65%, at least 70%, at least 75%, at least 80%, at least 85%, at least 90%, at least 95%, at least 98% or 100% sequence identity to the polypeptide shown in SEQ ID NO: 43.

One embodiment of the invention relates to a method of deep cleaning on an item, comprising the steps of:

a) contacting the item with a solution comprising an enzyme mixture comprising a dispersin, an alpha-amylase; and a cleaning component, wherein the cleaning component is selected from 0.1 to 50 wt% of at least one a surfactant; 0.5 to 30 wt% of at least one builder; and 0.01 to 20 wt% of at least one bleach component; and
b) and optionally rinsing the item, wherein the item is preferably a textile and

wherein the amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 30, and wherein the dispersin is selected from the group consisting of: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17.

One embodiment of the invention relates to a method of deep cleaning on an item, comprising the steps of:

a) contacting the item with a solution comprising an enzyme mixture comprising a dispersin, an alpha-amylase; and a cleaning component, wherein the cleaning component is selected from 0.1 to 50 wt% of at least one a surfactant; 0.5 to 30 wt% of at least one builder; and 0.01 to 20 wt% of at least one bleach component; and
b) and optionally rinsing the item, wherein the item is preferably a textile and

wherein the alpha-amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 31, and wherein the dispersin is selected from the group consisting of: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17.

One embodiment of the invention relates to a method of deep cleaning on an item, comprising the steps of:

a) contacting the item with a solution comprising an enzyme mixture comprising a dispersin, an alpha-amylase; and a cleaning component, wherein the cleaning component is selected from 0.1 to 50 wt% of at least one a surfactant; 0.5 to 30 wt% of at least one builder; and 0.01 to 20 wt% of at least one bleach component; and
b) and optionally rinsing the item, wherein the item is preferably a textile and

wherein the alpha-amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 32, and wherein the dispersin is selected from the group consisting of: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17.

One embodiment of the invention relates to a method of deep cleaning on an item, comprising the steps of:

a) contacting the item with a solution comprising an enzyme mixture comprising a dispersin, an alpha-amylase; and a cleaning component, wherein the cleaning component is selected from 0.1 to 50 wt% of at least one a surfactant; 0.5 to 30 wt% of at least one builder; and 0.01 to 20 wt% of at least one bleach component; and
b) and optionally rinsing the item, wherein the item is preferably a textile and

wherein the alpha-amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 33, and wherein the dispersin is selected from the group consisting of: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17.

One embodiment of the invention relates to a method of deep cleaning on an item, comprising the steps of:

a) contacting the item with a solution comprising an enzyme mixture comprising a dispersin, an alpha-amylase; and a cleaning component, wherein the cleaning component is selected from 0.1 to 50 wt% of at least one a surfactant; 0.5 to 30 wt% of at least one builder; and 0.01 to 20 wt% of at least one bleach component; and
b) and optionally rinsing the item, wherein the item is preferably a textile and

wherein the alpha-amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 43, and wherein the dispersin is selected from the group consisting of: a polypeptide having at least 60%, at least 70%, at least 75%, at least 80%, at least 85%, at least 90%, at least 95%, at least 98%, at least 99% or 100% sequence identity to the polypeptide shown in SEQ ID NO: 17

## Definitions

Biofilm is produced by any group of microorganisms in which cells stick to each other or stick to a surface, such as a textile, dishware or hard surface or another kind of surface. These adherent cells are frequently embedded within a self-produced matrix of extracellular polymeric substance (EPS). Biofilm EPS is a polymeric conglomeration generally composed of proteins, and polysaccharides, such as PNAG. Biofilms may form on living or non-living surfaces. The microbial cells growing in a biofilm are physiologically distinct from planktonic cells of the same organism, which, by contrast, are single-cells that may float or swim in a liquid medium. Bacteria living in a biofilm usually have significantly different properties from planktonic bacteria of the same species, as the dense and protected environment of the film allows them to cooperate and interact in various ways. One benefit of this environment for the microorganisms is increased resistance to detergents and antibiotics, as the dense extracellular matrix and the outer layer of cells protect the interior of

the community.

On laundry biofilm producing bacteria may e.g. be found among the following species: *Acinetobacter* sp., *Aeromicrobium* sp., *Brevundimonas* sp., *Microbacterium* sp., *Micrococcus luteus, Pseudomonas* sp., *Staphylococcus epidermidis,* and *Stenotrophomonas* sp. On hard surfaces biofilm producing bacteria may e.g. be found among the following species: *Acinetobacter* sp., *Aeromicrobium* sp., *Brevundimonas* sp., *Microbacterium* sp., *Micrococcus luteus, Pseudomonas* sp., *Staphylococcus epidermidis, Staphylococcus aureus* and *Stenotrophomonas* sp. In one aspect, the biofilm producing strain is *Brevundimonas* sp. In one aspect, the biofilm producing strain is *Pseudomonas alcaliphila* or *Pseudomonas fluorescens.* In one aspect, the biofilm producing strain is *Staphylococcus aureus.*

By the term "deep cleaning" is meant reduction, disruption or removal of components of organic matter, e.g. biofilm, such as polysaccharides, proteins, PNAG, soil or other components present in the organic matter.

Cleaning component: The cleaning component e.g. the detergent adjunct ingredient is different to the dispersin and carbohydrase. The precise nature of these additional cleaning components e.g. adjunct components, and levels of incorporation thereof, will depend on the physical form of the composition and the nature of the operation for which it is to be used. Suitable cleaning components e.g. adjunct materials include, but are not limited to the components described below such as surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric agents, clay soil removal/anti-redeposition agents, brighteners, suds suppressors, dyes, perfumes, structure elasticizing agents, fabric softeners, carriers, hydrotropes, builders and co-builders, fabric huing agents, anti-foaming agents, dispersants, processing aids, and/or pigments.

Cleaning Composition: The term "cleaning composition" refers to compositions that find use in the removal of undesired compounds from items to be cleaned, such as textiles. The detergent composition may be used to e.g. clean textiles for both household cleaning and industrial cleaning. The terms encompass any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; fabric fresheners; fabric softeners; and textile and laundry pre-spotters/pretreatment). In addition to containing the enzyme blend of the invention, the detergent formulation may contain one or more additional enzymes (such as proteases, lipases, cutinases, endoglucanases, xyloglucanases, pectinases, pectin lyases, xanthanases, peroxidases, haloperoxygenases and catalases or any mixture thereof), and/or detergent adjunct ingredients such as surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti-corrosion agents, enzyme inhibitors or stabilizers, enzyme activators, transferase(s), hydrolytic enzymes, oxido reductases, bluing agents and fluorescent dyes, antioxidants, and solubilizers.

The term "enzyme detergency benefit" is defined herein as the advantageous effect an enzyme may add to a detergent compared to the same detergent without the enzyme. Important detergency benefits which can be provided by enzymes are stain removal with no or very little visible soils after washing and/or cleaning, prevention or reduction of redeposition of soils released in the washing process (an effect that also is termed anti-redeposition), restoring fully or partly the whiteness of textiles which originally were white but after repeated use and wash have obtained a greyish or yellowish appearance (an effect that also is termed whitening). Textile care benefits, which are not directly related to catalytic stain removal or prevention of redeposition of soils, are also important for enzyme detergency benefits. Examples of such textile care benefits are prevention or reduction of dye transfer from one fabric to another fabric or another part of the same fabric (an effect that is also termed dye transfer inhibition or anti-backstaining), removal of protruding or broken fibers from a fabric surface to decrease pilling tendencies or remove already existing pills or fuzz (an effect that also is termed anti-pilling), improvement of the fabric-softness, colour clarification of the fabric and removal of particulate soils which are trapped in the fibers of the fabric or garment. Enzymatic bleaching is a further enzyme detergency benefit where the catalytic activity generally is used to catalyze the formation of bleaching components such as hydrogen peroxide or other peroxides. Textile care benefits, which are not directly related to catalytic stain removal or prevention of redeposition of soils, are also important for enzyme detergency benefits. Examples of such textile care benefits are prevention or reduction of dye transfer from one textile to another textile or another part of the same textile (an effect that is also termed dye transfer inhibition or anti-backstaining), removal of protruding or broken fibers from a textile surface to decrease pilling tendencies or remove already existing pills or fuzz (an effect that also is termed anti-pilling), improvement of the textile-softness, colour clarification of the textile and removal of particulate soils which are trapped in the fibers of the textile. Enzymatic bleaching is a further enzyme detergency benefit where the catalytic activity generally is used to catalyze the formation of bleaching component such as hydrogen peroxide or other peroxides or other bleaching species."

The term "hard surface cleaning" is defined herein as cleaning of hard surfaces wherein hard surfaces may include floors, tables, walls, roofs etc. as well as surfaces of hard objects such as cars (car wash) and dishes (dish wash). Dish washing includes but are not limited to cleaning of plates, cups, glasses, bowls, cutlery such as spoons, knives, forks, serving utensils, ceramics, plastics, metals, china, glass and acrylics.

The term "wash performance" is used as an enzyme's ability to remove stains present on the object to be cleaned

during e.g. wash or hard surface cleaning.

The term "whiteness" is defined herein as a greying, yellowing of a textile. Loss of whiteness may be due to removal of optical brighteners/hueing agents. Greying and yellowing can be due to soil redeposition, body soils, colouring from e.g. iron and copper ions or dye transfer. Whiteness might include one or several issues from the list below: colourant or dye effects; incomplete stain removal (e.g. body soils, sebum etc.); redeposition (greying, yellowing or other discolourations of the object) (removed soils reassociate with other parts of textile, soiled or unsoiled); chemical changes in textile during application; and clarification or brightening of colours.

The term "laundering" relates to both household laundering and industrial laundering and means the process of treating textiles with a solution containing a cleaning or detergent composition of the present invention. The laundering process can for example be carried out using e.g. a household or an industrial washing machine or can be carried out by hand.

By the term "malodor" is meant an odor which is not desired on clean items. The cleaned item should smell fresh and clean without malodors adhered to the item. One example of malodor is compounds with an unpleasant smell, which may be produced by microorganisms. Another example is unpleasant smells can be sweat or body odor adhered to an item which has been in contact with human or animal. Another example of malodor can be the odor from spices, which sticks to items for example curry or other exotic spices which smells strongly.

The term "mature polypeptide" means a polypeptide in its final form following translation and any post-translational modifications, such as N-terminal processing, C-terminal truncation, glycosylation, phosphorylation, etc.

The term "textile" means any textile material including yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens, denims, non-wovens, felts, yarns, and towelling. The textile may be cellulose based such as natural cellulosics, including cotton, flax/linen, jute, ramie, sisal or coir or manmade cellulosics (e.g. originating from wood pulp) including viscose/rayon, cellulose acetate fibers (tricell), lyocell or blends thereof. The textile or fabric may also be non-cellulose based such as natural polyamides including wool, camel, cashmere, mohair, rabbit and silk or synthetic polymers such as nylon, aramid, polyester, acrylic, polypropylene and spandex/elastane, or blends thereof as well as blends of cellulose based and non-cellulose based fibers. Examples of blends are blends of cotton and/or rayon/viscose with one or more companion material such as wool, synthetic fiber (e.g. polyamide fiber, acrylic fiber, polyester fiber, polyvinyl chloride fiber, polyurethane fiber, polyurea fiber, aramid fiber), and/or cellulose-containing fiber (e.g. rayon/viscose, ramie, flax/linen, jute, cellulose acetate fiber, lyocell). Fabric may be conventional washable laundry, for example stained household laundry. When the term fabric or garment is used it is intended to include the broader term textiles as well.

The term "variant" means a polypeptide having the activity of the parent or precursor polypeptide and comprising an alteration, i.e., a substitution, insertion, and/or deletion, at one or more (e.g., several) positions compared to the precursor or parent polypeptide. A substitution means replacement of the amino acid occupying a position with a different amino acid; a deletion means removal of the amino acid occupying a position; and an insertion means adding an amino acid adjacent to and immediately following the amino acid occupying a position.

Sequence identity: The relatedness between two amino acid sequences or between two nucleotide sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 6.6.0 or later. The parameters used are a gap open penalty of 10, a gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

Delta remission value (ΔRem): The terms "Delta remission" or "Delta remission value" are defined herein as the result of a reflectance or remission measurement at a certain wavelength which typically is 460 nm. The swatch is measured with one swatch of similar colour as background, preferably a swatch from a repetition wash. A swatch representing each swatch type is measured before the wash. The Delta remission is the remission value of the washed swatch minus the remission value of the unwashed swatch.

One way of measuring the wash performance is the Delta enzyme performance value (ΔRem enzyme value): The term "Delta enzyme remission value" is defined herein as the result of a reflectance or remission measurement at 460 nm. The swatch is measured with one swatch of similar colour as background, preferably a swatch from a repetition wash. A swatch representing each swatch type is measured before wash. The Delta enzyme remission is the remission value of the swatch washed in detergent with an enzyme present minus the remission value of a similar swatch washed in a

detergent without enzyme present.

**Examples**

<u>Assays</u>

**Assay I: testing of hexosaminidase activity**

The hexosaminidase activity of the dispersins was determined using 4-nitrophenyl N-acetyl-β-D-glucosaminide (Sigma-Aldrich) as substrate. The enzymatic reaction was performed in triplicates in a 96 well flat bottom polystyrene microtiter plate (Thermo Scientific) with the following conditions: 50 mM 2-(N-morpholino)ethanesulfonic acid pH 6 buffer, 1.5 mg/ml 4-nitrophenyl N-acetyl-β-D-glucosaminide and 20 μg/ml purified enzyme sample in a total reaction volume of 100 μl. Blank samples without polypeptide were run in parallel. The reactions were carried out at 37°C in a Thermomixer comfort (Eppendorf). After 10 minutes of incubation, 5 μl 1 M NaOH was added to each reaction mixture to stop the enzymatic reaction. The absorbance was read at 405 nm using a POLARstar Omega plate reader (BMG LABTECH) to estimate the formation of 4-nitrophenolate ion released because of enzymatic hydrolysis of the 4-nitrophenyl N-acetyl-β-D-glucosaminide substrate.

**Assay II: testing of mannanase activity**

Mannanase activity may be tested according to standard test procedures known in the art, such as by applying a solution to be tested to 4 mm diameter holes punched out in agar plates containing 0.2% AZCL galactomannan (carob), *i.e.* substrate for the assay of endo-1,4-beta-D-mannanase available as CatNo.I-AZGMA from the company Megazyme (Megazyme's Internet address: http://www.megazyme.com/Purchase/index.html).

**Assay III: testing of xyloglucanase activity**

The reaction involves endo hydrolysis of 1,4-beta-D-glucosidic linkages in xyloglucan. For purposes of the present invention, xyloglucanase activity is determined using AZCL-xyloglucan (from Megazyme) as the reaction substrate.

**Assay IV: testing of cellulase activity**

The term "cellulase activity" is defined herein as an enzyme catalyzed hydrolysis of 1,4-beta-D-glucosidic linkages in beta-1,4-glucan (cellulose). For purposes of the present invention, cellulase activity is determined using AZCL-HE-cellulose (from Megazyme) as the reaction substrate.

**Assay V: testing of amylase activity**

*1. PNP-G7 assay*

The alpha-amylase activity was determined by employing the pNP-G7 substrate (PNP-G7 the abbreviation for 4,6-ethylidene(G7)-p-nitrophenyl(G1)-α,D-maltoheptaoside, a blocked oligosaccharide which is cleaved by an endo-amylase, such as an alpha-amylase).

An antibody was diluted in Phosphate buffered saline (PBS) (0.010 M Phosphate buffer pH7.4, 0.0027M KCl, 0.14M NaCl) buffer to concentration of 10μg/ml. A maxisorp microtiter plate was coated with antibody by adding 100μl diluted antibody (10μg/ml) to each well and incubated for 1h at room temperature (RT) and mixing at 800 rpm. After incubation the microtiter plate was washed (using Bio-Tek ELx405 ELISA washer) with 3x 200μl Phosphate buffered saline with 0.05% Tween (PBST) (0.010 M Phosphate buffer pH7.4, 0.0027M KCl, 0.14M NaCl, 0.05% Tween 20) buffer.

Microtiter plates with the alpha-amylase variants culture broths were spun down and supernatants transferred to new microtiter plates and diluted 4x in PBST buffer. 100μl diluted supernatant was transferred to antibody coated maxisorp microtiter plate and incubated for 1h at RT and mixing at 800rpm. After incubation microtiter plates were washed in PBST buffer (3x 200μl, ELISA washer).

Upon the cleavage of the pNP-G7 substrate, the alpha-Glucosidase included in the kit used is digested and the hydrolysed substrate liberates a free pNP molecule which has a yellow color and thus can be measured by visible spectophometry at Abs=405nm (400-420 nm.). Kits containing pNP-G7 substrate and alpha-Glucosidase are manufactured by Roche/Hitachi (cat. No.11876473). 100μl pNP-G7 substrate was added to all wells and mixed for 1 minute before measuring absorbance at 405nm. The slope (absorbance per minute) is determined and only the linear range of

curve is used.

The slope of the time dependent absorption-curve is directly proportional to the activity of the alpha-amylase in question under the given set of conditions.

The specific alpha-amylase activity may also be determined by other activity assays, such as amylazyme activity assay, Phadebas activity assay, or reducing sugar activity assay as described below.

*2. Amylazyme activity assay*

Amylazyme activity assay (from Megazyme, Ireland): An Amylazyme tablet includes interlinked amylose polymers that are in the form of globular microspheres that are insoluble in water. A blue dye is covalently bound to these microspheres. The interlinked amylose polymers in the microsphere are degraded at a speed that is proportional to the alpha-amylase activity. When the alpha-amylase degrades the amylose polymers, the released blue dye is water soluble and concentration of dye can be determined by measuring absorbance at 650nm. The concentration of blue is proportional to the alpha-amylase activity in the sample.

The amylase sample to be analysed is diluted in activity buffer with the desired pH. One substrate tablet is suspended in 5mL activity buffer and mixed on magnetic stirrer. During mixing of substrate transfer 150μl to microtiter plate (MTP). Add 30μl diluted amylase sample to 150μl substrate and mix. Incubate for 15 minutes at 37°C. The reaction is stopped by adding 30μl 1M NaOH and mix. Centrifuge MTP for 5 minutes at 4000xg. Transfer 100μl to new MTP and measure absorbance at 620nm.

The amylase sample should be diluted so that the absorbance at 650nm is between 0 and 2.2 and is within the linear range of the activity assay.

*3. Phadebas assay*

A Phadebas tablet (from for example Magle Life Sciences, Lund, Sweden) includes interlinked starch polymers that are in the form of globular microspheres that are insoluble in water. A blue dye is covalently bound to these microspheres. The interlinked starch polymers in the microsphere are degraded at a speed that is proportional to the alpha-amylase activity. When the alpha-amylase degrades the starch polymers, the released blue dye is water soluble and concentration of dye can be determined by measuring absorbance at 650nm. The concentration of blue is proportional to the alpha-amylase activity in the sample.

The amylase sample to be analysed is diluted in activity buffer with the desired pH. One substrate tablet is suspended in 5mL activity buffer and mixed on magnetic stirrer. During mixing of substrate transfer 150μl to microtiter plate (MTP). Add 30μl diluted amylase sample to 150μl substrate and mix. Incubate for 15 minutes at 37°C. The reaction is stopped by adding 30μl 1M NaOH and mix. Centrifuge MTP for 5 minutes at 4000xg. Transfer 100μl to new MTP and measure absorbance at 620nm.

The measured absorbance is directly proportional to the specific activity (activity/mg of pure alpha-amylase protein) of the alpha-amylase in question under the given set of conditions.

*4. Reducing sugar activity assay*

Number of reducing ends formed by the alpha-amylase hydrolysing the alpha-1,4-glycosidic linkages in starch is determined by reaction with p-Hydroxybenzoic acid hydrazide (PHBAH). After reaction with PHBAH the number of reducing ends can be measured by absorbance at 405nm and the concentration of reducing ends is proportional to the alpha-amylase activity in the sample.

The corns starch substrate (3mg/ml) is solubilised by cooking for 5 minutes in milliQ water and cooled down before assay. For the stop solution prepare a Ka-Na-tartrate/NaOH solution (K-Na-tartrate (Merck 8087) 50g/l, NaOH 20g/l) and prepare freshly the stop solution by adding p-Hydroxybenzoic acid hydrazide (PHBAH, Sigma H9882) to Ka-Na-tartrate/NaOH solution to 15mg/ml.

In PCR-MTP 50μl activity buffer is mixed with 50μl substrate. Add 50μl diluted enzyme and mix. Incubate at the desired temperature in PCR machine for 5 minutes. Reaction is stopped by adding 75μl stop solution (Ka-Na-tartrate/-NaOH/PHBAH). Incubate in PCR machine for 10 minutes at 95°C. Transfer 150μl to new MTP and measure absorbance at 405nm.

The measured absorbance is directly proportional to the specific activity (activity/mg of pure alpha-amylase protein) of the alpha-amylase in question under the given set of conditions.

**Example 1**

**Composition of model detergent A (liquid)**

Ingredients: 12% LAS, 11% AEO Biosoft N25-7 (NI), 5% AEOS (SLES), 6% MPG (monopropylene glycol), 3% ethanol, 3% TEA, 2.75% coco soap, 2.75% soya soap, 2% glycerol, 2% sodium hydroxide, 2% sodium citrate, 1% sodium formate, 0.2% DTMPA and 0.2% PCA copoly(acrylic acid/maleic acid) (all percentages are w/w)

**Extraction of crude biofilm EPS (extracellular polymeric substances) from *Pseudomonas fluorescens***

A crude EPS (extracellular polymeric substances) extract was prepared from *Pseudomonas fluorescens* as follows: *P.fluorescens* was restreaked on Tryptic Soy Agar and incubated at ambient temperature. The strain was inoculated in TSB and incubated over night at 20°C. After propagation, the culture was diluted (1:100) in M63 supplemented medium (15mM $(NH_4)_2SO_4$, 100mM $KH_2PO_4$ , 1.8μM $FeSO_4$, 1mM $MgSO_4$.7H2O, 0.4% (w/v) glycerol, 0.2% (w/v) Casamino acids and 0.0001% (w/v) Thiamine), added to a Corning® CellBIND® 225cm$^2$ Angled Neck Cell Culture Flasks with Vent Cap (200ml per flask) and incubated statically for 3 days at 20°C. The biofilm culture was subsequently pelleted by centrifugation (10min, 8000g, 25°C), and the cells were resuspended in 3M NaCl and incubated for 30min at 30°C to extract the surface-associated EPS. The EPS-containing supernatant obtained after centrifugation (10min, 5000g, 25°C) was stored at -20°C until further use.

**Synergistic effect between dispersin and amylase on deep-cleaning in liquid model detergent on mixed soil swatches**

Textile finishing, e.g. ironing starch, may attract dirt and other environmental components, including microbial soil. This may in turn lead to undesirable issues with the items, such as increased greying of the textile or malodor formation. To simulate this problem, a soil swatch composed of a finishing agent (ironing starch) and microbial soil (crude biofilm EPS) was prepared as follows: Decarbonated ironing starch (Sterling Polish Company A/S, DK) was mixed with a crude biofilm EPS from *Pseudomonas fluorescens* (see description above) in a 1:1 ratio, along with 0.5 mg/ml iron(III) oxide nano-powder (544884; Sigma-Aldrich). Clean textile swatches (2-cm, wfk20A (Wfk-Testgewebe GmbH)) were then soaked in this mixture and the swatches were dried over night at ambient temperature.

For testing deep-cleaning wash performance, the swatches were placed in 50 mL test tubes and 10 mL of wash liquor (15°dH water with 3.33g/L liquid model A detergent) and 0.1 μg/ml dispersin and/or 3 μg/ml amylase was added to each tube. Washes without enzyme were included as controls. The test tubes were placed in a Stuart rotator and incubated for 1 hour at 30°C at 20rpm. The wash liquor was then removed, and the swatches were rinsed twice with 15°dH water and dried on filter paper over night. The remission ($REM^{460nm}$) values were measured using a Datacolor 800V and are displayed in table 1. Delta values ($REM^{460nm}_{(swatches\ washed\ with\ enzyme)}$ - $REM^{460nm}_{(swatches\ washed\ without\ enzyme)}$) and the wash performance synergies, $WP_{synergy}$ ($\Delta REM^{460nm}{}_{(blend)}$ - $\Delta REM^{460nm}{}_{(sum\ of\ individual\ enzyme\ treatments)}$) are also indicated.

Table 1. Synergistic effect of dispersin and amylase on cleaning in model A detergent on soil swatches.

| Swatch | Enzyme concentration (μg/ml) | Average Rem460nm | WP (delta Rem460nm) | $WP_{syn}$ |
|---|---|---|---|---|
| Mixed stain, no enzyme | 0.0 | 54.2 | | |
| Mixed stain, SEQ ID NO 17 | 0.1 | 55.7 | 1.5 | |
| Mixed stain, SEQ ID NO 30* | 3 | 56.2 | 2.0 | |
| Mixed stain, SEQ ID NO 33 | 3 | 57.6 | 3.4 | |
| Mixed stain, SEQ ID NO 43 | 3 | 56.5 | 2.3 | |
| Mixed stain, SEQ ID NO 17 + SEQ ID NO 30* | 0.1 + 3 | 65.3 | 11.1 | 7.6 |
| Mixed stain, SEQ ID NO 17 + SEQ ID NO 33 | 0.1 + 3 | 63.6 | 9.4 | 4.6 |
| Mixed stain, SEQ ID NO 17 + SEQ ID NO 43 | 0.1 + 3 | 64.8 | 10.6 | 6.9 |
| *SEQ ID NO 30 with the mutations (R118K, D183*, G184*, N195F, R320K, R458K), where D183* and G184* means deletion of amino acids D (Asp) and G(Gly) at positions 183 and 184 respectively and R118K means replacement of R (Arg) with K (Lys) and so forth. | | | | |

As seen in table 1 an enzyme combination comprising both dispersin and amylase provides superior cleaning e.g. deep-cleaning properties in model A detergent as compared to the individual enzymes. Given that the wash performance of the enzyme blend (WP (blend)) exceed the sum of the performances seen for of the individual enzymes (WP (enzyme 1) + WP(enzyme 2)), i.e. $WP_{syn} > 0$, this clearly suggests that there is a synergistic effect between the two enzymes on the deep-cleaning properties in model A. This also suggests that these types of textile soils can interact with each other and impede deep-cleaning of the other soil type, either through the formation of complex macromolecular interactions or by physically shielding it.

## Claims

1. A cleaning composition comprising a dispersin, at least one carbohydrase and a cleaning component, wherein the dispersin is a polypeptide having hexosaminidase activity and at least 60% sequence identity to the polypeptide of SEQ ID NO: 17, and wherein the carbohydrase is an alpha-amylase.

2. The cleaning composition of claim 1, wherein the dispersin is a polypeptide having hexosaminidase activity and at least 70% sequence identity to the polypeptide shown in SEQ ID NO: 17.

3. The cleaning composition of claim 1 or 2, wherein the dispersin is a polypeptide having hexosaminidase activity and at least 75%, at least 80%, at least 85%, at least 90%, at least 95%, at least 98%, at least 99% or 100% sequence identity to the polypeptide shown in SEQ ID NO: 17.

4. The cleaning composition of any of claims 1-3, wherein the amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 33.

5. The cleaning composition of any of claims 1-3, wherein the amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 30.

6. The cleaning composition of any of claims 1-3, wherein the amylase is a polypeptide having at least 60%, at least 65%, at least 70%, at least 75%, at least 80%, at least 85%, at least 90%, at least 95%, at least 98% or 100% sequence identity to the polypeptide shown in SEQ ID NO: 43.

7. The cleaning composition according to any of the preceding claims, wherein the amount of dispersin in the composition is from 0.01 to 1000 ppm and the amount of carbohydrase is from 0.01 to 1000 ppm.

8. The cleaning composition according to any of the preceding claims, wherein the cleaning component is selected from surfactants, preferably anionic and/or nonionic, builders and bleach components.

9. Use of a cleaning composition according to any of claims 1 to 8 for deep cleaning of an item, wherein the item is a textile or a surface.

10. A method of formulating a cleaning composition according to any of claims 1 to 8, the method comprising adding a dispersin, a carbohydrase and at least one cleaning component, wherein the dispersin is a polypeptide having hexosaminidase activity and at least 60% sequence identity to the polypeptide of SEQ ID NO: 17, and wherein the carbohydrase is an alpha-amylase.

11. A kit intended for cleaning, wherein the kit comprises a solution of an enzyme mixture comprising a dispersin and a carbohydrase, wherein the dispersin is a polypeptide having hexosaminidase activity and at least 60% sequence identity to the polypeptide of SEQ ID NO: 17, and wherein the carbohydrase is an alpha-amylase.

12. A method of cleaning of an item, comprising the steps of:

    a) contacting the item with a cleaning composition according to any of claims 1 to 8; and optionally,
    b) rinsing the item.

13. The method of claim 12, wherein the item is a textile.

**Patentansprüche**

1. Reinigungszusammensetzung, umfassend ein Dispersin, mindestens eine Carbohydrase und eine Reinigungskomponente, wobei das Dispersin ein Polypeptid mit Hexosaminidase-Aktivität und mindestens 60 % Sequenzidentität zu dem Polypeptid gemäß SEQ ID NO: 17 ist und wobei die Carbohydrase eine Alpha-Amylase ist.

2. Reinigungszusammensetzung nach Anspruch 1, wobei das Dispersin ein Polypeptid mit Hexosaminidase-Aktivität und mindestens 70 % Sequenzidentität zu dem in SEQ ID NO: 17 gezeigten Polypeptid ist.

3. Reinigungszusammensetzung nach Anspruch 1 oder 2, wobei das Dispersin ein Polypeptid ist, das Hexosaminidase-Aktivität und mindestens 75 %, mindestens 80 %, mindestens 85 %, mindestens 90 %, mindestens 95 %, mindestens 98 %, mindestens 99 % oder 100 % Sequenzidentität mit dem in SEQ ID NO: 17 gezeigten Polypeptid aufweist.

4. Reinigungszusammensetzung nach einem der Ansprüche 1 bis 3, wobei die Amylase ein Polypeptid ist, das mindestens 60 %, mindestens 65 %, mindestens 70 %, mindestens 75 %, mindestens 80 %, mindestens 85 %, mindestens 90 %, mindestens 95 %, mindestens 98 % oder 100 % Sequenzidentität mit dem in SEQ ID NO: 33 gezeigten Polypeptid aufweist.

5. Reinigungszusammensetzung nach einem der Ansprüche 1 bis 3, wobei die Amylase ein Polypeptid ist, das mindestens 60 %, mindestens 65 %, mindestens 70 %, mindestens 75 %, mindestens 80 %, mindestens 85 % mindestens 90 %, mindestens 95 %, mindestens 98 % oder 100 % Sequenzidentität mit dem in SEQ ID NO: 30 gezeigten Polypeptid aufweist.

6. Reinigungszusammensetzung nach einem der Ansprüche 1 bis 3, wobei die Amylase ein Polypeptid ist, das mindestens 60 %, mindestens 65 %, mindestens 70 %, mindestens 75 %, mindestens 80 %, mindestens 85 %, mindestens 90 %, mindestens 95 %, mindestens 98 % oder 100 % Sequenzidentität mit dem in SEQ ID NO: 43 gezeigten Polypeptid aufweist.

7. Reinigungszusammensetzung gemäß einem der vorstehenden Ansprüche, wobei die Menge an Dispersin in der Zusammensetzung 0,01 bis 1000 ppm und die Menge an Carbohydrase 0,01 bis 1000 ppm beträgt.

8. Reinigungszusammensetzung gemäß einem der vorstehenden Ansprüche, wobei die Reinigungskomponente ausgewählt ist aus Tensiden, vorzugsweise anionischen und/oder nichtionischen, Gerüststoffen und Bleichkomponenten.

9. Verwendung einer Reinigungszusammensetzung gemäß einem der Ansprüche 1 bis 8 zur Tiefenreinigung eines Gegenstands, wobei der Gegenstand ein Textil oder eine Oberfläche ist.

10. Verfahren zur Formulierung einer Reinigungszusammensetzung gemäß einem der Ansprüche 1 bis 8, wobei das Verfahren die Zugabe eines Dispersins, einer Carbohydrase und mindestens einer Reinigungskomponente umfasst, wobei das Dispersin ein Polypeptid ist, das Hexosaminidase-Aktivität und mindestens 60 % Sequenzidentität zu dem Polypeptid gemäß SEQ ID NO: 17 aufweist und wobei die Carbohydrase eine Alpha-Amylase ist.

11. Kit zum Reinigen, wobei das Kit eine Lösung eines Enzymgemischs umfasst, das ein Dispersin und eine Carbohydrase umfasst, wobei das Dispersin ein Polypeptid ist, das Hexosaminidase-Aktivität und mindestens 60 % Sequenzidentität mit dem Polypeptid gemäß SEQ ID NO: 17 aufweist und wobei die Carbohydrase eine Alpha-Amylase ist.

12. Verfahren zum Reinigen eines Gegenstands, umfassend die Schritte:

   a) In-Kontakt-Bringen des Gegenstands mit einer Reinigungszusammensetzung gemäß einem der Ansprüche 1 bis 8; und optional
   b) Spülen des Gegenstands.

13. Verfahren nach Anspruch 12, wobei der Gegenstand ein Textil ist.

**Revendications**

1. Composition nettoyante comprenant une dispersine, au moins une carbohydrase et un composant nettoyant, dans laquelle la dispersine est un polypeptide ayant une activité hexosaminidase et une identité de séquence d'au moins 60 % avec le polypeptide de SEQ ID NO : 17, et dans laquelle la carbohydrase est une alpha-amylase.

2. Composition de nettoyage selon la revendication 1, dans laquelle la dispersine est un polypeptide ayant une activité hexosaminidase et une identité de séquence d'au moins 70 % avec le polypeptide représenté dans la SEQ ID N° 17.

3. Composition nettoyante selon la revendication 1 ou 2, dans laquelle la dispersine est un polypeptide ayant une activité hexosaminidase et au moins 75 %, au moins 80 %, au moins 85 %, au moins 90 %, au moins 95 %, au moins 98 %, au moins 99 % ou 100 % d'identité de séquence avec le polypeptide représenté dans SEQ ID NO : 17.

4. La composition nettoyante de l'une quelconque des revendications 1 à 3, dans laquelle l'amylase est un polypeptide ayant au moins 60 %, au moins 65 %, au moins 70 %, au moins 75 %, au moins 80 %, au moins 85 %, au moins 90 %, au moins 95 %, au moins 98 % ou 100 % d'identité de séquence avec le polypeptide représenté dans SEQ ID NO : 33.

5. La composition nettoyante de l'une quelconque des revendications 1 à 3, dans laquelle l'amylase est un polypeptide ayant au moins 60 %, au moins 65 %, au moins 70 %, au moins 75 %, au moins 80 %, au moins 85 %, au moins 90 %, au moins 95 %, au moins 98 % ou 100 % d'identité de séquence avec le polypeptide représenté dans SEQ ID NO : 30.

6. Composition de nettoyage selon l'une quelconque des revendications 1 à 3, dans laquelle l'amylase est un polypeptide ayant au moins 60 %, au moins 65 %, au moins 70 %, au moins 75 %, au moins 80 %, au moins 85 %, au moins 90 %, au moins 95 %, au moins 98 % ou 100 % d'identité de séquence avec le polypeptide représenté dans SEQ ID NO : 43.

7. Composition nettoyante selon l'une quelconque des revendications précédentes, dans laquelle la quantité de dispersine dans la composition est de 0,01 à 1 000 ppm et la quantité de carbohydrase est de 0,01 à 1 000 ppm.

8. Composition nettoyante selon l'une quelconque des revendications précédentes, dans laquelle le composant nettoyant est choisi parmi les tensioactifs, de préférence anioniques et/ou non ioniques, les adjuvants et les composants de blanchiment.

9. Utilisation d'une composition nettoyante selon l'une quelconque des revendications 1 à 8 pour le nettoyage en profondeur d'un article, l'article étant un textile ou une surface.

10. Procédé de formulation d'une composition nettoyante selon l'une quelconque des revendications 1 à 8, le procédé comprenant l'ajout d'une dispersine, d'une carbohydrase et d'au moins un composant nettoyant, dans lequel la dispersine est un polypeptide ayant une activité hexosaminidase et une identité de séquence d'au moins 60 % avec le polypeptide de SEQ ID NO : 17, et dans lequel la carbohydrase est une alpha-amylase.

11. Kit destiné au nettoyage, dans lequel le kit comprend une solution d'un mélange d'enzymes comprenant une dispersine et une carbohydrase, dans lequel la dispersine est un polypeptide ayant une activité hexosaminidase et au moins 60 % d'identité de séquence avec le polypeptide de SEQ ID NO : 17, et dans lequel la carbohydrase est une alpha-amylase.

12. Procédé de nettoyage d'un article, comprenant les étapes consistant à :

    a) mettre en contact l'article avec une composition nettoyante selon l'une quelconque des revendications 1 à 8 ; et éventuellement,
    b) rincer l'article.

13. Procédé selon la revendication 12, dans lequel l'article est un textile.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3088506 A
- WO 2018002194 A
- WO 1999064619 A
- WO 2017021515 A
- WO 2017021516 A
- WO 2017021517 A
- WO 2017021518 A
- WO 2015040159 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- WO 99001544 A
- WO 2002099091 A
- WO 2001062903 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 13184577 A
- WO 10104675 A
- WO 2011098531 A
- WO 2013001078 A
- WO 2013001087 A
- WO 2017186936 A
- WO 2017186937 A
- WO 2017186943 A
- WO 09102854 A
- US 5977053 A
- WO 9817767 A
- EP 624154 A
- WO 2007087258 A
- WO 2007087244 A
- WO 2007087259 A
- EP 1867708 A, Vitamin K
- WO 2007087242 A
- WO 9426860 A
- WO 9426859 A
- WO 2006130575 A
- WO 200503274 A
- WO 200503275 A
- WO 200503276 A
- EP 1876226 A
- WO 2007087257 A
- WO 2007087243 A
- US 7262042 B
- WO 09021867 A
- WO 9318140 A
- WO 01016285 A
- WO 002016547 A
- WO 9425583 A
- WO 05040372 A
- WO 05052161 A
- WO 05052146 A
- WO 9523221 A
- WO 9221760 A
- EP 1921147 A
- EP 1921148 A
- WO 07044993 A
- WO 8906279 A
- WO 9219729 A
- WO 96034946 A
- WO 9820115 A
- WO 9820116 A
- WO 99011768 A
- WO 0144452 A
- WO 03006602 A
- WO 0403186 A
- WO 04041979 A
- WO 07006305 A
- WO 11036263 A
- WO 11036264 A
- WO 2016001449 A
- WO 2004067737 A
- US 5352604 A
- EP 258068 A
- EP 305216 A

- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A
- WO 12137147 A
- EP 407225 A
- WO 9205249 A
- WO 9401541 A
- WO 9425578 A
- WO 9514783 A
- WO 9530744 A
- WO 9535381 A
- WO 9522615 A
- WO 9600292 A
- WO 9704079 A
- WO 9707202 A
- WO 0034450 A
- WO 0060063 A
- WO 0192502 A
- WO 0787508 A
- WO 09109500 A
- WO 10111143 A
- WO 0556782 A
- WO 0967279 A
- WO 10100028 A
- WO 9324618 A
- WO 9510602 A
- WO 9815257 A
- WO 9201046 A
- JP 2238885 A
- WO 9708325 A
- WO 9533836 A
- WO 2009087523 A
- WO 2007138054 A
- WO 2006108856 A
- WO 2006113314 A
- EP 1867808 A
- WO 2003040279 A
- EP 2169040 A
- US 20090011970 A1
- US 4106991 A
- US 4661452 A
- GB 1483591 A
- EP 238216 A
- WO 2013188331 A
- WO 9307263 A
- WO 9723606 A
- WO 0001793 A
- WO 2006034710 A
- WO 9932595 A

**Non-patent literature cited in the description**

- **SIEZEN et al.** *Protein Engng.*, 1991, vol. 4, 719-737
- **SIEZEN et al.** *Protein Science*, 1997, vol. 6, 501-523
- Powdered Detergents, Surfactant science series. Marcel Dekker, Inc, vol. 71
- Powdered Detergents, Surfactant science series. Marcel Dekker, Inc., vol. 71
- **C. E. CAPES**. Handbook of Powder Technology. Elsevier, 1980, vol. 1
- **NEEDLEMAN** ; **WUNSCH**. *J. Mol. Biol.*, 1970, vol. 48, 443-453
- **RICE et al.** EMBOSS: The European Molecular Biology Open Software Suite. *Trends Genet.*, 2000, vol. 16, 276-277